# EUROPEAN PATENT APPLICATION

(11) **EP 4 728 993 A2**
(43) Date of publication of application: **22.04.2026**
(21) Application number: 25227086.3
(22) Date of filing: 21.12.2020
(51) Int. Cl.: A61B 8/08

(54) **CATHETER ULTRASOUND DEVICES AND METHODS FOR ASSESSING TARGETED TISSUE**

(30) Priority: 10.01.2020 US 202062959837 P
(62) Divisional of application: 20838625.0
(71) Applicant: Edwards Lifesciences Innovation (Israel) Ltd., 3079892 Caesarea (IL)
(72) Inventor: SHARON, Assaf, 6291606 Tel Aviv (IL); KEIDAR, Yaron, 5550729 Kiryat Ono (IL); GALON, Aviv, 6249247 Tel Aviv (IL); SHEPS, Tal, 5401453 Givat Shmuel (IL); HERMAN, Yaron, 3780800 Givat Ada (IL)
(74) Representative: Venner, Julia Ann

(57) **Abstract**

An implant (20) comprises a wall (28) that surrounds a lumen (44). A delivery tool (8) comprises a catheter (22), a control assembly (64), and an ultrasound tool (36). The catheter is transluminally advanceable into a subject, and has a distal opening. The implant can be delivered via the catheter. The control assembly can advance at least a portion (70) of the wall out of the distal opening, and can steer the catheter to place the portion against a site of a tissue of the subject, the site disposed distally from the portion and opposite the distal opening. The ultrasound tool can (i) position an ultrasound transceiver within the lumen and facing the portion, and (ii) facilitate imaging of the site by transmitting ultrasound energy through the portion. An anchor driver can anchor the implant to the tissue by driving an anchor through the portion and into the site.

## Description

### CROSS-REFERENCES TO RELATED APPLICATIONS

The present application claims priority from US Provisional Patent Application 62/959,837 to Sharon et al., filed January 10, 2020, and entitled "Catheter ultrasound devices and methods for assessing targeted tissue," which is incorporated herein by reference.

### BACKGROUND

Dilation of the annulus of a heart valve, such as that caused by ischemic heart disease, prevents the valve leaflets from fully coapting when the valve is closed. Regurgitation of blood from the ventricle into the atrium results in increased total stroke volume and decreased cardiac output, and ultimate weakening of the ventricle secondary to a volume overload and a pressure overload of the atrium.

### SUMMARY OF THE INVENTION

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure may be included in the examples summarized here.

Examples of apparatuses, systems, techniques, methods, etc. for implanting an implant at a tissue of a subject are described herein.

For some applications, an extracorporeal control assembly is used to steer a distal part of a catheter, such that the distal opening is disposed opposite a site of the tissue. For some applications, a portion of a wall of the implant can be placed against the site, and an ultrasound tool can be used to assess the site of the tissue, e.g., by transmitting ultrasound energy through the portion of the wall and to the site and detecting ultrasound energy reflected from the site.

For some applications, an anchor driver can be used to anchor the implant to the tissue by driving a tissue anchor through the portion of the wall and into the site of the tissue.

For some applications, aspects include an anchor channel defining a longitudinal cavity, and a distal region including a distal aperture. The anchor driver can be advanced, while coupled to the tissue anchor, distally through the longitudinal cavity to the implant, while the distal region is disposed within the implant, and the distal aperture is disposed at the portion of the wall. For example, the portion of the wall can be anchored to the site of the tissue while using the anchor channel to hold the portion of the wall against the site of the tissue.

For some applications, aspects include positioning an ultrasound transceiver of the ultrasound tool within a lumen of the implant and facing the portion of the wall.

For example, the ultrasound transceiver can be shaped as a ring defining a passage therethrough. The tissue anchor can be advanced, while coupled to the anchor driver, through the passage, reaching the portion of the wall. In this way, the anchor driver can be advanced through the cavity and through the passage, while the ultrasound transceiver remains in the lumen of the implant.

Optionally, the anchor driver and the ultrasound tool can be dimensioned such that only one of the anchor driver or the ultrasound transceiver can be disposed within the longitudinal cavity at a given time. For such applications, the anchor driver can be configured to be advanceable through the longitudinal cavity to the implant only while the ultrasound tool is not disposed through the longitudinal cavity, and therefore the ultrasound tool and the anchor driver are advanced and withdrawn in turn, in order to assess the tissue site and then anchor to the tissue site. For some such applications, the anchor channel is used to hold the portion of the wall against the site of the tissue during withdrawal of the ultrasound tool from the cavity and subsequent advancement of the anchor driver through the cavity to the implant.

For some applications, more than one anchor is used to anchor a corresponding more than one portion of the wall of the implant to a corresponding more than one site of the tissue. For some such applications, ultrasound-based assessment is performed for each of the sites.

For some applications, aspects include using a connector to relay data indicative of the reflected ultrasound energy detected by the ultrasound transceiver, from the ultrasound transceiver to an ultrasound controller. The ultrasound controller can be used to analyze the data.

For some applications, the tissue can include tissue of an annulus of a heart, the implant can include an annuloplasty structure. For some applications, anchoring the implant to the tissue can comprise anchoring a portion of a wall of the annuloplasty structure to the tissue of the annulus. For some such applications, the annuloplasty structure can include a contraction member (e.g., contraction wire, contraction line, etc.) extending along the sleeve of the annuloplasty structure, and an adjustment mechanism of the annuloplasty structure can be configured to contract the sleeve by tensioning the contraction member.

For some applications, the implant and/or annuloplasty structure can be a design without a wall or inner lumen or a sleeveless design; for example, the implant and/or annuloplasty structure can comprise and/or consist of a contraction member (e.g., contraction wire, contraction line, etc.) connected to (e.g., threaded through, etc.) multiple anchors.

For some applications, aspects include placing an indicator wire in a blood vessel of the heart of a subject and generating a physical field with respect to the indicator wire by driving an electric current through the indicator wire. For example, the physical field can comprise an electrostatic field, an electromagnetic field, an electric field, or a magnetic field.

For some applications, aspects include using an anchor-delivery system to deliver a tissue anchor toward a portion of the tissue adjacent to the blood vessel. Often, the anchor-delivery system includes a sensing element at a portion of the anchor-delivery system. For some applications, the sensing element is used to determine a position of a portion of the anchor-delivery system by bringing the portion of the anchor-delivery system within range of the indicator wire. For example, the position of the portion can be determined by measuring a change in the physical field.

For some applications, aspects include placing an indicator wire in a blood vessel of the heart of a subject, and generating an ultrasonic field using an ultrasonic sensing element by bringing the portion of the anchor-delivery system within range of the indicator wire. For example, the anchor-delivery system can include an ultrasonic sensing element at a portion of the anchor-delivery system. For some such applications, the position of the portion is determined by measuring a change in the ultrasonic field.

For some applications, aspects include calculating a distance between the portion of the anchor-delivery system and a portion of the indicator wire. For example, calculating the distance can include comparing the distance to a predetermined threshold.

For some applications, aspects include moving the portion of the anchor-delivery system through a lumen of an annuloplasty structure and deploying a tissue anchor into the portion of tissue from within the lumen of the annuloplasty structure. For example, the tissue anchor can be deployed into the portion of tissue responsively to comparing of the distance to the predetermined threshold.

There is therefore provided, in accordance with an application, a system and/or apparatus for use with a tissue of a subject, the system and/or apparatus including an implant and a delivery tool. For some applications, the implant comprises a contraction member (e.g., contraction wire, contraction line, etc.) connected to (e.g., threaded through, directly connected, operatively connected/coupled, etc.) multiple anchors. For some applications, the implant includes a wall that surrounds a lumen. For some applications, the implant does not include a lumen and/or wall.

The delivery tool includes a catheter, transluminally advanceable to the tissue and having a distal part that includes a distal opening. The implant can be configured to be delivered to the tissue via the catheter. The delivery tool also includes an extracorporeal control assembly. The control assembly can be configured to advance at least a portion of the implant (e.g., a portion of the wall, a length of the implant, a portion of a contraction member, an anchor, etc.) out of the distal opening. The control assembly can also be operably coupled to the distal part to steer the distal part to place the portion of the implant (e.g., portion of the wall, length of the implant, etc.) against a site of the tissue, the site disposed distally from the portion of the implant (e.g., portion of the wall, length of the implant, etc.) and opposite the distal opening.

For some applications, the delivery tool further includes an ultrasound tool. The ultrasound tool can be advanceable within the catheter and can include an ultrasound transceiver at a distal end. For some applications, the ultrasound tool is configured to position the ultrasound transceiver in a position that is facing the portion of the implant and facilitate imaging of the site by transmitting ultrasound energy through the portion of the implant and into the site. For some applications, where the portion of the implant is a portion of the wall of an implant with a lumen, the ultrasound tool is configured to position the ultrasound transceiver in a position that is within the lumen of the implant and facing the portion of the wall of the implant, and facilitate imaging of the site by transmitting ultrasound energy through the portion of the wall (e.g., from within the lumen) and into the site.

The system and/or apparatus can further include a tissue anchor; and an anchor driver. The anchor driver can be configured to anchor the implant to the tissue by driving the tissue anchor through the portion of the wall and into the site.

In an application, the catheter is configured to be transfemorally and transseptally advanceable to the tissue.

In an application, a distal part of the catheter is radiopaque.

In an application, the anchor driver is configured to advance the tissue anchor through the catheter to the implant while the implant is disposed at the tissue.

In an application, the ultrasound transceiver is configured to detect reflected ultrasound energy, the reflected ultrasound energy being a portion of the ultrasound energy that is transmitted by the ultrasound transceiver, and that is reflected from the site. The ultrasound tool can include an ultrasound controller including circuitry and a user interface, the ultrasound controller configured to facilitate analysis of the reflected ultrasound energy detected by the ultrasound transceiver. The ultrasound tool can include a connector configured to relay, from the ultrasound transceiver to the ultrasound controller, data indicative of the reflected ultrasound energy detected by the ultrasound transceiver.

In an application, the ultrasound transceiver is configured to detect reflected ultrasound energy that is reflected from the site through the portion of the wall.

In an application, the delivery tool includes an anchor channel, the anchor channel defining a longitudinal cavity ending at a distal aperture. The anchor channel can extend through the catheter such that a distal region of the anchor channel is disposed at the portion of the implant (e.g., within the lumen of the implant, against a side of the implant, adjacent the implant, etc.). For some applications, the distal region includes the distal aperture. For some applications, the anchor channel is advanceable within the catheter and a lumen of the implant.

In an application, the anchor channel is configured to extend through the catheter such that a distal region of the anchor channel is disposed within the lumen, the distal region including the distal aperture, and the anchor channel facilitates positioning of the portion of the wall at the site.

In an application, the anchor driver is advanceable, while coupled to the tissue anchor, through the longitudinal cavity to the site, so as to advance the tissue anchor into the site, and is removable from the longitudinal cavity (whether coupled to the tissue anchor or decoupled therefrom), via a proximal opening of the catheter.

In an application, the anchor driver is advanceable, while coupled to the tissue anchor, through the longitudinal cavity to the implant, so as to advance the tissue anchor into the lumen of the implant, in a manner in which the tissue anchor reaches the portion of the wall, and removable from the longitudinal cavity, via a proximal opening of the catheter.

In an application, the ultrasound transceiver is advanceable through the longitudinal cavity into the lumen of the implant such that the ultrasound transceiver is facing the portion of the wall and removable from the longitudinal cavity, through the proximal opening of the catheter.

In an application, the anchor driver is advanceable through the longitudinal cavity to the implant only while the ultrasound transceiver is not disposed through the longitudinal cavity.

In an application, the ultrasound tool includes the anchor channel, the ultrasound transceiver is disposed at the distal region of the anchor channel, and the anchor channel is configured to position the ultrasound transceiver in a position at the site. The position at the site can be a position that is within the lumen of the implant and facing the portion of the wall of the implant.

In an application, the ultrasound transceiver is shaped as a ring, the ring defining a passage therethrough.

In an application, the anchor driver is advanceable, while coupled to the tissue anchor, through the longitudinal cavity to the implant, so as to advance the tissue anchor through the passage, in a manner in which the tissue anchor reaches the portion of the implant (e.g., the portion of the wall of the implant, etc.).

In an application, the implant includes an annuloplasty structure. In an application, the annuloplasty structure includes a sleeve defined by the wall.

In an application, the annuloplasty structure includes a contraction member and an actuatable adjustment mechanism, and the adjustment mechanism is configured to, when actuated, apply tension to the contraction member, and the contraction member is configured to adjust a length of the annuloplasty structure when the contraction member is tensioned by actuating the adjustment mechanism.

There is further provided, in accordance with an application, method for implanting an implant at a tissue of a subject, the method including transluminally advancing the implant (or a portion thereof) to the tissue using a delivery tool that includes a catheter, the catheter having a distal part that includes a distal opening. The method further includes positioning an ultrasound transceiver of an ultrasound tool adjacent or proximate the portion of the implant at/near a site of the tissue. The method further includes assessing the site of the tissue, by using the ultrasound tool to transmit ultrasound energy to the site and detect reflected ultrasound energy reflected from the site.

The method can include using an extracorporeal control assembly that is operably coupled to the distal part of the catheter to steer the distal part of the catheter, such that the distal opening is disposed at the site of the tissue. The control assembly can also be used to hold (and the method can include holding), against the site of the tissue, a portion of the implant that is exposed out of the distal opening.

For some applications, the method includes assessing the site of the tissue, by using the ultrasound tool to transmit ultrasound energy through the portion of the implant and to the site and detect reflected ultrasound energy reflected from the site.

The method can also include: subsequently, using the anchor driver, anchoring the implant to the tissue by driving a tissue anchor through the portion of the implant and into the site of the tissue. For some applications, the portion of the implant can be portion of a wall of the implant, a portion of a sleeve of the implant, a portion of a side of the implant, a length of the implant, an area of the implant, a component of the implant, a tissue anchor (e.g., all or a portion thereof), etc.

For some applications, the tissue includes tissue of an annulus of a heart of a subject, the implant includes an annuloplasty structure, and anchoring the implant to the tissue includes anchoring a portion of the annuloplasty structure to the tissue of the annulus.

For some applications, the portion of the implant is a portion of a wall of the implant and detecting reflected ultrasound energy reflected from the site of the tissue includes detecting reflected ultrasound energy reflected back through the portion of the wall. For some applications, the implant includes a lumen circumscribed by the wall of the implant and the ultrasound tool or ultrasound transceiver thereof is positioned inside the lumen of the implant when transmitting ultrasound energy into the site and/or detecting ultrasound energy reflected from the site.

For some applications, the anchor driver is coaxial with the ultrasound tool when anchoring the implant to the tissue. For some applications, the tissue anchor is coaxial with a distal region and/or distal end of the ultrasound tool and/or ultrasound transceiver when anchoring the implant to the tissue.

For some applications, the method further comprises using the ultrasound tool and the anchor driver simultaneously while anchoring the implant to the tissue. For some applications, the method further comprises continuing to assess the site of the tissue using the ultrasound tool while anchoring the implant to the tissue by driving the tissue anchor into the site of the tissue.

For some applications, the ultrasound transceiver of the ultrasound tool is disposed at a distal region of the ultrasound tool, holding the portion of the implant against the site of the tissue includes, using the ultrasound tool, exerting a pushing force against the portion (which, in some applications, can optionally be done from within the lumen), and driving the tissue anchor includes driving the tissue anchor through a passage defined by the ultrasound transceiver.

In an application, transluminally advancing the implant to the tissue using the catheter includes transfemorally advancing the implant to the tissue using the catheter. In an application, transluminally advancing the implant to the tissue using the catheter includes transseptally advancing the implant to the tissue using the catheter.

For some applications, the method includes positioning an anchor channel such that a distal region of the anchor channel is disposed at or near the site, the anchor channel defining a longitudinal cavity, and the distal region including a distal aperture, and the distal aperture is disposed at the portion of the implant. The method can further include advancing the anchor driver, while coupled to the tissue anchor, distally through the longitudinal cavity to the site, while the distal aperture is disposed at or near the site.

For some applications, anchoring the implant to the tissue includes anchoring the portion of the implant to the site of the tissue, while using the anchor channel to hold the portion of the implant at the site of the tissue.

For some applications, positioning the ultrasound transceiver includes advancing the ultrasound tool distally through the longitudinal cavity, while the distal aperture is disposed at the site.

For some applications, the method includes, prior to advancing the anchor driver distally through the longitudinal cavity to the implant, withdrawing the ultrasound transceiver from the longitudinal cavity, through a proximal opening of the catheter, while holding the portion of the implant at the site of the tissue using the anchor channel.

For some applications, the ultrasound tool includes a connector and an ultrasound controller that includes circuitry and a user interface, and the method includes using the connector, relaying data indicative of the reflected ultrasound energy detected by the ultrasound transceiver, from the ultrasound transceiver to the ultrasound controller, and using the circuitry of the ultrasound controller, analyzing the reflected ultrasound energy.

For some applications, analyzing the reflected ultrasound energy includes operating a data analysis program in the circuitry. In an application, analyzing the reflected ultrasound energy includes evaluating an image displayed on the user interface.

For some applications, the tissue anchor is a first tissue anchor, the portion of the implant is a first portion of the implant, the site is a first site of the tissue, and driving the tissue anchor into the site of the tissue includes driving the first tissue anchor into the first site of the tissue. This can be done while (or as) the first tissue anchor is connected/coupled to the first portion of the implant. Further, anchoring the implant to the tissue can include, subsequently to driving the first tissue anchor into the first site of the tissue, driving a second tissue anchor into a second site of the tissue. This can be done while (or as) the second tissue anchor is connected/coupled to the second portion of the implant.

For some applications, the first site is at a left fibrous trigone of the annulus and anchoring the implant to the tissue includes driving the first tissue anchor into the first site at the left fibrous trigone.

For some applications, the first site is at a right fibrous trigone of the annulus and anchoring the implant to the tissue includes driving the first tissue anchor into the first site at the right fibrous trigone.

For some applications, the implant comprises an annuloplasty structure, and the annuloplasty structure includes a contraction member (e.g., a contraction wire, a contraction suture, a contraction line, etc.). In an application, the method includes adjusting a size of an annulus of a native valve by tensioning the contraction member. In an application, the method includes adjusting a length of the annuloplasty structure by tensioning the contraction member.

For some applications, the implant includes an annuloplasty structure, a wall of the implant being shaped to define a sleeve of the annuloplasty structure, and the annuloplasty structure including a contraction member extending along at least a portion of the sleeve. In an application, the method includes adjusting a size of an annulus of a native valve by tensioning the contraction member. The method can include adjusting a length of the annuloplasty structure by tensioning the contraction member.

For some applications, adjusting the size of the annulus of the native valve by tensioning the contraction member includes tensioning the contraction member by actuating an adjustment mechanism.

For some applications, adjusting the length of the structure by tensioning the contraction member includes tensioning the contraction member by actuating an adjustment mechanism.

For some applications, the adjustment mechanism includes a rotatable spool coupled to the contraction member and tensioning the contraction member by actuating the adjustment mechanism includes rotating the spool.

For some applications, the method includes (i) subsequently to driving the first tissue anchor into the first site of the tissue, and (ii) prior to driving the second tissue anchor into the second site of the tissue: steering the distal part of the catheter, such that the distal opening is disposed at or near the second site of the tissue; holding the second portion of the implant at or near the second site of the tissue; assessing the second site of the tissue, by using the ultrasound tool to: both transmit ultrasound energy into the second site and detect reflected ultrasound energy reflected from the second site.

For some applications, driving the second tissue anchor into the second site of the tissue includes driving the second tissue anchor into the second site of the tissue while the ultrasound transceiver remains within the catheter and is at or near the second portion of the implant.

For some applications, assessing the second site of the tissue includes assessing the second site of the tissue without removing the ultrasound transceiver from the catheter and/or from proximate the native heart valve between assessing the first site of the tissue and assessing the second site of the tissue.

For some applications, the method includes: (i) subsequently to assessing the first site of the tissue, and (ii) prior to driving the first tissue anchor into the first site of the tissue, withdrawing the ultrasound transceiver from the lumen of the implant; and (i) subsequently to driving the first tissue anchor into the first site of the tissue, and (ii) prior to assessing the second site of the tissue:
withdrawing the anchor driver from the implant; and
subsequently, advancing the ultrasound transceiver into the lumen of the implant such that the ultrasound transceiver faces the second site.

The above method(s) can be performed on a living animal or on a simulation, such as on a cadaver, cadaver heart, simulator (e.g. with the body parts, heart, tissue, etc. being simulated), etc.

There is further provided, in accordance with an application, method for implanting an implant at a tissue of a subject, the implant including a wall that surrounds a lumen, the method including transluminally advancing the implant to the tissue using a delivery tool that includes a catheter, the catheter having a distal part that includes a distal opening. The method including using an extracorporeal control assembly that is operably coupled to the distal part of the catheter to steer the distal part of the catheter, such that the distal opening is disposed at or opposite a site of the tissue. In some applications, the control assembly can be used to hold (and the method can include holding), against the site of the tissue, a portion of the implant that is exposed out of the distal opening.

The method further includes positioning an ultrasound transceiver of an ultrasound tool within the lumen of the implant and facing the portion of the wall. The method further includes assessing the site of the tissue, by using the ultrasound tool to transmit ultrasound energy through the portion of the wall and into the site and detect reflected ultrasound energy reflected from the site.

The method can also include: subsequently, using the anchor driver, anchoring the implant to the tissue by driving a tissue anchor through the portion of the wall and into the site of the tissue.

In an application the tissue includes tissue of an annulus of a heart of a subject, the implant includes an annuloplasty structure, the wall being a wall of the annuloplasty structure, and anchoring the implant to the tissue includes anchoring a portion of the wall of the annuloplasty structure to the tissue of the annulus.

In an application, detecting reflected ultrasound energy reflected from the site of the tissue includes detecting reflected ultrasound energy reflected back through the portion of the wall.

In an application, the ultrasound transceiver of the ultrasound tool is disposed at a distal region of the ultrasound tool, holding the portion of the wall against the site of the tissue includes, using the ultrasound tool, exerting a pushing force against the portion from within the lumen, and driving the tissue anchor includes driving the tissue anchor through a passage defined by the ultrasound transceiver.

In an application, transluminally advancing the implant to the tissue using the catheter includes transfemorally advancing the implant to the tissue using the catheter.

In an application, transluminally advancing the implant to the tissue using the catheter includes transseptally advancing the implant to the tissue using the catheter.

In an application, the method includes positioning an anchor channel such that a distal region of the anchor channel is disposed within the lumen, the anchor channel defining a longitudinal cavity, and the distal region including a distal aperture, and the distal aperture is disposed at the portion of the wall. The method can further include advancing the anchor driver, while coupled to the tissue anchor, distally through the longitudinal cavity to the implant, while the distal aperture is disposed at the portion of the wall.

In an application, anchoring the implant to the tissue includes anchoring the portion of the wall to the site of the tissue, while using the anchor channel to hold the portion of the wall against the site of the tissue.

In an application, positioning the ultrasound transceiver includes advancing the ultrasound tool distally through the longitudinal cavity, while the distal aperture is disposed at the portion of the wall.

In an application, the method includes, prior to advancing the anchor driver distally through the longitudinal cavity to the implant, withdrawing the ultrasound transceiver from the longitudinal cavity, through a proximal opening of the catheter, while holding the portion of the wall against the site of the tissue using the anchor channel.

In an application, the ultrasound tool includes a connector and an ultrasound controller that includes circuitry and a user interface, and the method includes using the connector, relaying data indicative of the reflected ultrasound energy detected by the ultrasound transceiver, from the ultrasound transceiver to the ultrasound controller, and using the circuitry of the ultrasound controller, analyzing the reflected ultrasound energy.

In an application, analyzing the reflected ultrasound energy includes operating a data analysis program in the circuitry. In an application, analyzing the reflected ultrasound energy includes evaluating an image displayed on the user interface.

In an application, the tissue anchor is a first tissue anchor, the portion of the wall is a first portion of the wall, the site is a first site of the tissue, and driving the tissue anchor through the portion of the wall and into the site of the tissue includes driving the first tissue anchor through the first portion of the wall and into the first site of the tissue. Further, anchoring the implant to the tissue can include, subsequently to driving the first tissue anchor through the first portion of the wall and into the first site of the tissue, driving a second tissue anchor through a second portion of the wall and into a second site of the tissue.

In an application, the first site is at a left fibrous trigone of the annulus and anchoring the implant to the tissue includes driving the first tissue anchor through the first portion of the wall and into the first site at the left fibrous trigone.

In an application, the first site is at a right fibrous trigone of the annulus and anchoring the implant to the tissue includes driving the first tissue anchor through the first portion of the wall and into the first site at the right fibrous trigone.

In an application, the implant includes an annuloplasty structure, the wall being shaped to define a sleeve of the annuloplasty structure, and the annuloplasty structure including a contraction member extending along at least a portion of the sleeve, and the method includes adjusting a length of the structure by tensioning the contraction member.

In an application, adjusting the length of the structure by tensioning the contraction member includes tensioning the contraction member by actuating an adjustment mechanism.

In an application, the adjustment mechanism includes a rotatable spool coupled to the contraction member, and tensioning the contraction member by actuating the adjustment mechanism includes rotating the spool.

In an application, the method includes (i) subsequently to driving the first tissue anchor through the first portion of the wall and into the first site of the tissue, and (ii) prior to driving the second tissue anchor through the second portion of the wall and into the second site of the tissue: steering the distal part of the catheter, such that the distal opening is disposed opposite the second site of the tissue; holding the second portion of the wall against the second site of the tissue; assessing the second site of the tissue, by using the ultrasound tool to: both transmit ultrasound energy through the second portion of the wall and into the second site and detect reflected ultrasound energy reflected from the second site.

In an application, driving the second tissue anchor through the second portion of the wall and into the second site of the tissue includes driving the second tissue anchor through the second portion of the wall and into the second site of the tissue while the ultrasound transceiver remains within the lumen of the implant, and facing the second portion of the wall.

In an application, assessing the second site of the tissue includes assessing the second site of the tissue without removing the ultrasound transceiver from within the lumen of the implant between assessing the first site of the tissue and assessing the second site of the tissue.

In an application, the method includes: (i) subsequently to assessing the first site of the tissue, and (ii) prior to driving the first tissue anchor through the first portion of the wall, withdrawing the ultrasound transceiver from the lumen of the implant; and (i) subsequently to driving the first tissue anchor through the first portion of the wall and into the first site of the tissue, and (ii) prior to assessing the second site of the tissue:
withdrawing the anchor driver from the implant; and
subsequently, advancing the ultrasound transceiver into the lumen of the implant such that the ultrasound transceiver faces the second portion of the wall.

The above method(s) can be performed on a living animal or on a simulation, such as on a cadaver, cadaver heart, simulator (e.g. with the body parts, heart, tissue, etc. being simulated), etc.

There is further provided, in accordance with an application, a method, including:
placing an indicator wire in a blood vessel of a heart of a subject;
generating a physical field with respect to the indicator wire by driving an electric current through the indicator wire;
using an anchor-delivery system, delivering a tissue anchor toward a portion of tissue of the heart adjacent to the blood vessel, the anchor-delivery system including a sensing element at a portion of the anchor-delivery system; and
determining a position of the portion of the anchor-delivery system by:
   measuring a change in the physical field by bringing the portion of the anchor-delivery system within range of the indicator wire; and
   by the measuring, calculating a distance between the portion of the anchor-delivery system and a portion of the indicator wire.

In an application, placing the indicator wire in the blood vessel includes placing the indicator wire in a coronary artery of the heart.

In an application, placing the indicator wire in the blood vessel includes placing the indicator wire in a coronary vein of the heart.

In an application, generating the physical field includes generating an electrostatic field.

In an application, generating the physical field includes generating an electromagnetic field.

In an application, generating the physical field includes generating an electric field.

In an application, generating the physical field includes generating a magnetic field.

In an application, the sensing element includes an ultrasonic sensor.

In an application, delivering the tissue anchor includes implanting an annuloplasty structure at an annulus of the heart of the subject using the tissue anchor.

In an application, the annuloplasty structure comprises a contraction member (e.g., a contraction wire, a contraction line, a contraction suture, etc.) and one or more tissue anchors.

In an application, the annuloplasty structure is shaped so as to define a lumen, and delivering the tissue anchor includes moving the portion of the anchor-delivery system through the lumen of the annuloplasty structure and deploying the tissue anchor into the portion of tissue from within the lumen of the annuloplasty structure.

In an application, the annuloplasty structure does not include a lumen or sleeve.

In an application, calculating the distance between the portion of the anchor-delivery system and the portion of the indicator wire includes comparing the distance to a predetermined threshold.

In an application, delivering the tissue anchor includes deploying the tissue anchor into the portion of tissue responsively to the comparing of the distance to a predetermined threshold.

In an application, delivering the tissue anchor includes deploying the tissue anchor into the portion of tissue responsively to determining that the distance is below the predetermined threshold.

In an application, the tissue anchor defines a first tissue anchor, and the method includes:
subsequently to the deploying of the tissue anchor into the portion of tissue, moving the portion of the anchor-delivery system to a second portion of tissue by determining the position of the portion of the anchor delivery system; and
deploying the second tissue anchor into the second portion of tissue responsively to the comparing of the distance to the predetermined threshold.

In an application, delivering the second tissue anchor includes deploying the second tissue anchor into the second portion of tissue responsively to determining that the distance is below the predetermined threshold.

The above method(s) can be performed on a living animal or on a simulation, such as on a cadaver, cadaver heart, simulator (e.g. with the body parts, heart, tissue, etc. being simulated), etc.

There is further provided, in accordance with an application, a method, including:
placing an indicator wire in a blood vessel of a heart of a subject;
using an anchor-delivery system, delivering a tissue anchor toward a portion of tissue of the heart adjacent to the blood vessel, the anchor-delivery system including an ultrasonic sensing element at a portion of the anchor-delivery system; and
determining a position of the portion of the anchor-delivery system by:
   measuring a change in an ultrasonic field generated by the ultrasonic sensing element by bringing the portion of the anchor-delivery system within range of the indicator wire; and
   by the measuring, calculating a distance between the portion of the anchor-delivery system and a portion of the indicator wire.

In an application, placing the indicator wire in the blood vessel includes placing the indicator wire in a coronary artery of the heart.

In an application, placing the indicator wire in the blood vessel includes placing the indicator wire in a coronary vein of the heart.

In an application, the portion of tissue includes tissue of an annulus of the subject, the method includes advancing an annuloplasty structure to the annulus, the annuloplasty structure shaped so as to define a lumen therethrough, and delivering the tissue anchor to the portion of tissue includes delivering the tissue anchor from within the lumen of the annuloplasty structure and to a portion of tissue of the annulus.

In an application, delivering the tissue anchor includes implanting an annuloplasty structure at an annulus of the heart of the subject using the tissue anchor.

In an application, the annuloplasty structure comprises a contraction member (e.g., a contraction wire, a contraction line, a contraction suture, etc.) and one or more tissue anchors.

In an application, the annuloplasty structure is shaped so as to define a lumen and delivering the tissue anchor includes moving the portion of the anchor-delivery system through the lumen of the annuloplasty structure and deploying the tissue anchor into the portion of tissue from within the lumen of the annuloplasty structure.

In an application, the annuloplasty structure does not include a lumen or sleeve.

In an application, calculating the distance between the portion of the anchor-delivery system and the portion of the indicator wire includes comparing the distance to a predetermined threshold.

In an application, delivering the tissue anchor includes deploying the tissue anchor into the portion of tissue responsively to the comparing of the distance to a predetermined threshold.

In an application, delivering the tissue anchor includes deploying the tissue anchor into the portion of tissue responsively to determining that the distance is below the predetermined threshold.

In an application, the tissue anchor defines a first tissue anchor, and the method includes:
subsequently to the deploying of the tissue anchor into the portion of tissue, moving the portion of the anchor-delivery system to a second portion of tissue by determining the position of the portion of the anchor delivery system; and
deploying the second tissue anchor into the second portion of tissue responsively to the comparing of the distance to the predetermined threshold.

In an application, delivering the second tissue anchor includes deploying the second tissue anchor into the second portion of tissue responsively to determining that the distance is below the predetermined threshold.

The above method(s) can be performed on a living animal or on a simulation, such as on a cadaver, cadaver heart, simulator (e.g. with the body parts, heart, tissue, etc. being simulated), etc.

The present invention will be more fully understood from the following detailed description of applications thereof, taken together with the drawings, in which:

### BRIEF DESCRIPTION OF THE DRAWINGS

Fig. 1 is a schematic illustration showing a multi-component system comprising an example implant and an example delivery tool for delivering the implant to a tissue of a subject, in accordance with some applications;
Figs. 2A-M are schematic illustrations showing an example delivery tool being used to deploy an example implant at cardiac tissue of a heart, in accordance with some applications;
Figs. 3A-F are schematic illustrations showing various positions of an example ultrasound tool with respect to cardiac tissue, in accordance with some applications;
Fig. 4 is a schematic illustration showing a multi-component system comprising an example implant, and an example delivery tool for delivering the implant to the tissue of a subject, in accordance with some applications;
Figs. 5A-J are schematic illustrations showing an example delivery tool being used to deploy an example implant at cardiac tissue of the heart, in accordance with some applications;
Fig. 6 is a flow chart that schematically illustrates at least some steps of an example method for implanting an implant at a tissue, in accordance with some applications; and
Figs. 7A-B are schematic illustrations of an example system for detecting a location of a portion of an anchor-delivery system, in accordance with some applications.

### DETAILED DESCRIPTION OF EMBODIMENTS

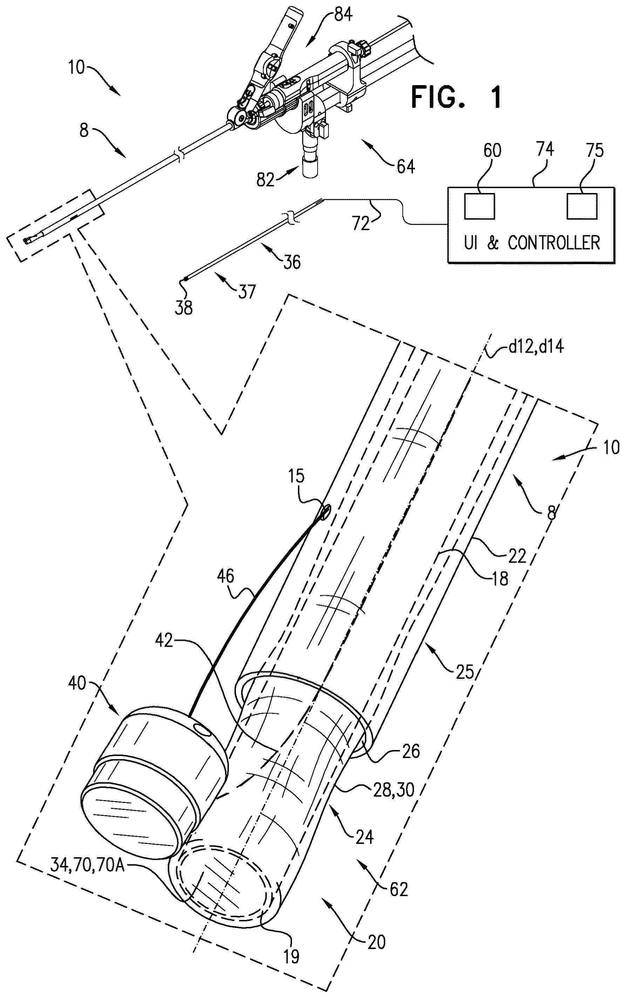

Reference is made to Fig. 1, which is a schematic illustration of a multi-component system 10 comprising an implant 20, and a delivery tool 8 for delivering the implant to a tissue of a subject, in accordance with some applications. Throughout this application, the implant of system 10 is described as comprising an annuloplasty structure. One example annuloplasty structure referenced herein is one that includes a sleeve and/or a lumen. However, it is to be noted that, for some applications, the systems, apparatuses, methods, techniques, etc. described herein can be used to facilitate implantation of other annuloplasty structures and/or other types of implants, mutatis mutandis.

Implant 20 is an example of one possible implant, one that comprises a wall 28 that surrounds a lumen 44. For example, and as shown, implant 20 can comprise a sleeve 30, and wall 28 can define a tubular lateral wall that surrounds lumen 44. For some applications, and as shown, lumen 44 is an elongate lumen (e.g., the interior of implant 20 is shaped as an elongate lumen). For some applications, wall 28 defines a distal end wall 34 of implant 20 (e.g., in addition to the tubular lateral wall).

Fig. 1 shows a distal portion of system 10 such that implant 20 is disposed partially within a catheter 22 of tool 8, with a distal portion 62 of the implant exposed from a distal opening 26 of the catheter. For some applications, implant 20 is an elongate implant defining an implant axis d12 therealong. Where implant 20 comprises a sleeve 30, the implant axis can be considered a sleeve axis. Often, and as shown, distal portion 62 is disposed along axis d12. Catheter 22 is configured to be transluminally/transvascularly advanced (e.g. transfemorally and/or transseptally advanced) to the tissue at which implant 20 is to be implanted and used to deliver the implant to the tissue via distal opening 26.

Extracorporeal control assembly 64 is operably coupled to a distal part 25 of catheter 22 to steer the distal part, e.g., to facilitate advancing of the catheter to the tissue. For example, and as shown, extracorporeal control assembly 64 can comprise one or more handles 82 and steering control elements 84, configured to facilitate steering of catheter 22 (e.g. distal part 25 thereof) by an operator, and/or manipulation of implant 20.

The term "steerable" as used herein (including the specification and the claims) means actively steerable, e.g., by using an extracorporeal controller to effect bending. (This is in contrast to a flexible but non-steerable element, which may bend in response to encountering forces during advancement through the body of the subject.) In this way, distal part 25 can be steered to place one or more portions 70 (e.g., a wall or portion(s) thereof, a sleeve or portion(s) thereof, a contraction member or portion(s) thereof, an anchor(s) or portion(s) thereof, a side or portion(s) thereof, , a component(s) or portion(s) thereof, a length, a width, an area, etc.) of implant 20 against one or more respective sites 68 of the tissue. Each of the respective sites can be (i) disposed distally from the respective portion of the implant, and (ii) opposite distal opening 26.

For some applications, and as shown, implant 20 is anchored to the tissue by anchoring a plurality of anchors 52 to a corresponding plurality of anchoring sites 68 of the tissue (e.g. a first site 68a, a second site 68b, etc.). For some applications, portions 70 of the implant (e.g. a first portion 70a, a second portion 70b, etc.) are anchored to a corresponding plurality of anchoring sites 68 of the tissue (e.g. a first site 68a, a second site 68b, etc.), e.g., by anchoring a corresponding plurality of anchors 52 at the corresponding plurality of portions of the implant.

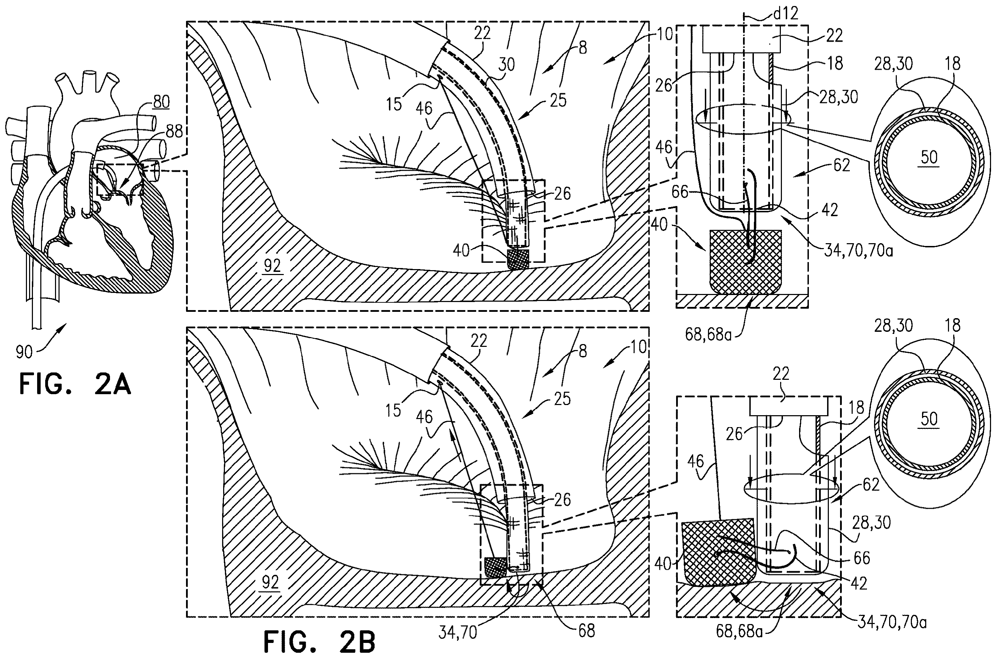

For some applications, first portion 70a can be located within distal portion 62 of sleeve 30. For some applications, and as shown in Figs. 2A-K, first portion 70a is located at distal end wall 34 of sleeve 30.

For some applications, catheter 22 is configured to be transfemorally and transseptally advanceable to the tissue. This is not meant to exclude other means and/or approaches for advancing catheter 22 to the tissue.

For some applications, distal part 25 of catheter 22 is radiopaque and/or includes one or more discrete radiopaque markers to facilitate positioning of distal part 25 at, against, or near a desired site of the tissue. This can also be used to facilitate placement of a portion 70 of the wall against site 68 of the tissue.

For some applications, delivery tool 8 further comprises an anchor channel 18 defining a longitudinal cavity 50 disposed along a channel axis d14. Channel 18 is advanceable (e.g. slidable, screwable, etc.) within catheter 22.

Channel 18 can be advanceable within both catheter 22 and lumen 44 of implant 20. For such applications, sleeve 30 is often concentric with channel 18 (e.g., such that axis d12 is colinear with axis d14).

For some applications, prior to implantation (e.g., within the operating theater or in an adjacent room), a distal region 24 of channel 18 is loaded into sleeve 30, and implant 20 is loaded into catheter 22. In this way, distal region 24, including a distal aperture 19 of channel 18, can be advanced distally within catheter 22 from a proximal opening of catheter 22 to distal part 25 of the catheter, such that distal region 24 is disposed within lumen 44 of implant 20.

As shown, channel 18 extends through catheter 22, such that distal region 24, including a distal aperture 19 of anchor channel 18, is disposed within lumen 44 of implant 20, and longitudinal cavity 50 ends at the distal aperture.

For some applications, anchor channel 18 is configured to facilitate directed deployment of implant 20, e.g., as described, mutatis mutandis, in US Patent Application Publication 2018/0049475 to Iflah et al., which is incorporated herein by reference. For some applications, sleeve 30 comprises a flexible material, such that the sleeve (e.g., each portion 70 thereof) is moved into position by moving anchor channel 18, e.g., by steering catheter 22. For example, sleeve 30 can comprise a braided fabric mesh, e.g., comprising polyethylene terephthalate (such as Dacron (TM)). As described in more detail hereinbelow, the position of anchor channel 18 with respect to implant 20 may define each portion 70. For example, in an initial position of anchor channel 18 within lumen 44, a first portion 70a can be defined opposite distal aperture 19 (e.g., by virtue of it being the portion of the implant that will receive a first anchor that will be advanced via cavity 50), and in a second position of the anchor channel within the lumen, a second portion 70b can be defined opposite the distal aperture (e.g., by virtue of it being the portion of the implant that will receive a second anchor that will be advanced via the lumen).

For some applications, implant 20 comprises a flexible elongated contraction member 42 (e.g., a contraction wire, contraction line, contraction suture, etc.). For some applications, and as shown, implant 20 comprises an annuloplasty structure that comprises the flexible elongated contraction member 42. The contraction member 42 can be configured to extend along at least a portion of sleeve 30, the portion of the sleeve along which member 42 extends thereby being defined as a contracting portion of the sleeve. Though for some applications, the contraction member is used without a sleeve, e.g., the contraction member may be connected and/or coupled directly to anchors (e.g., passing through an eyelet or other portion thereof, or otherwise connected/coupled thereto), and contraction of the contraction member can pull the tissue anchors and thereby the annulus into a different (e.g., smaller) shape/configuration without requiring a sleeve or even fabric. The contraction member(s) described anywhere herein can comprise a wire, a ribbon, a rope, or a band, and can comprise a flexible and/or superelastic material, e.g., nitinol, polyester, stainless steel, or cobalt chrome. For some applications, the contraction member or wire comprises a radiopaque material. For some applications, the contraction member comprises a braided polyester suture (e.g., Ticron). For some applications, the contraction member is coated with polytetrafluoroethylene (PTFE). For some applications, the contraction member comprises a plurality of wires that are intertwined to form a rope structure.

For some applications (including, for example, where implant 20 comprises annuloplasty structure), and as shown, the structure further comprises an actuatable adjustment mechanism 40, which facilitates contracting and expanding of the structure. Adjustment mechanism 40 is coupled to the contraction member at an end portion of the contraction member. When actuated, adjustment mechanism 40 applies tension to the contraction member, which in turn adjusts a length of the implant and/or annuloplasty structure. For some applications, adjustment mechanism 40 can be coupled by a coupling 66 (e.g., a suture, sutures, adhesive, fasteners, etc.) to sleeve 30. For some applications, adjustment mechanism 40 is coupled to an outer, lateral surface of sleeve 30.

For some applications in which implant 20 comprises adjustment mechanism 40, system 10 comprises a flexible, longitudinal guide member 46 (e.g., a wire, line, guide, etc.) coupled to a portion of the adjustment mechanism. Guide member 46 extends from adjustment mechanism 40 and proximally through catheter 22 (e.g., through a parallel side-lumen of the catheter) and has a proximal end that is accessible from outside the body of the subject.

For some applications in which implant 20 comprises contraction member 42, the implant does not comprise a discrete adjustment mechanism, and instead the contraction member is tensioned by pulling proximally, e.g., using an adjustment tool or tools, e.g., while providing a reference force against the implant. For such applications, a locking member or lock (e.g., clip, fastener, staple, crimper, clamp, etc.) can be locked to contraction member 42 in order to lock in the applied tension. For some such applications, the locking member is a component of the implant, and is therefore introduced into the subject simultaneously with the rest of the implant. Optionally, the locking member can be introduced subsequent to the introduction of the implant into the subject, e.g., with the adjustment tool. Implants without a discrete adjustment mechanism as described here can include a sleeve and/or a lumen, or can be sleeveless or without a lumen (e.g., some can comprise a contraction member and tissue anchors without a sleeve or fabric, etc.)

Reference is made to Figs. 2A-M, which are schematic illustrations showing tool 8 being used to deploy implant 20 at cardiac tissue 92 of a heart 90 of a subject, in accordance with some applications.

System 10 is described herein below as being used, inter alia, for advancing implant 20 to cardiac tissue 92 and for anchoring the implant to the cardiac tissue. For some applications, tissue 92 includes tissue of an annulus 88 of heart 90. For some applications, implant 20 comprises an annuloplasty structure, and for some such applications, anchoring the implant to tissue 92 comprises anchoring wall 28 of the annuloplasty structure to the tissue of annulus 88. However, this is not meant to exclude use of system 10 for deploying alternate implants to annulus 88 and/or to other tissues, mutatis mutandis.

Implant 20 is generally transluminally delivered to cardiac tissue 92 via a catheter 22. While a transfemoral transseptal approach to the mitral valve is shown in Fig. 2A, the scope of the invention includes alternate approaches to the mitral valve, to other locations in (e.g., valves of) the heart, and to other locations in the body.

For some applications, implant 20 is delivered by advancing catheter 22 with the implant already disposed therein. Optionally, catheter 22 can be advanced first, and implant 20 can be subsequently advanced through the catheter. For some such applications, implant 20 can be advanced with distal region 24 of anchor channel 18 already disposed in the interior of implant 20. For some such applications, anchor channel 18 can be introduced into the interior after advancement of implant 20.

Distal part 25 of catheter 22 can be steered, using extracorporeal control assembly 64, to a location opposite a first site 68a of cardiac tissue 92, such that the first site is disposed: (i) distally from first portion 70a of the implant (e.g., distally of wall of sleeve 30, distally from end wall 34, etc.), and (ii) opposite distal opening 26 of the catheter (Figs. 2A-B). Similarly, for some applications, first site 68a can be defined by virtue of it being disposed opposite first portion 70a.

For some applications, sites 68 can be pre-defined prior to advancing implant 20 and/or anchors 52 to the tissue. For example, sites 68 can be defined in relation to anatomical landmarks (e.g. a fibrous trigone or commissures) of heart 90. Optionally, each site 68 can be defined in an ad-hoc manner during implantation, simply as the site to which the implant and/or tissue anchor is to be anchored.

For some applications, portions 70 can be pre-defined prior to advancing implant 20 to the tissue. For example, portions 70 can be defined in relation to a dimension (e.g. a length) of implant 20, and/or can be discrete portions or components of the implant. Optionally, implant 20 itself may not define portions 70 as discrete portion, but instead each portion 70 may be defined, in an ad-hoc manner during implantation, simply as the portion and/or component of the implant that is to be anchored to the tissue (e.g., the portion of the implant that is opposite distal aperture 19 of anchor channel 18).

For some applications, and as shown, the first part of implant 20 to be anchored to the tissue (e.g., to first site 68a) is distal end wall 34 of sleeve 30. That is, for some applications, first portion 70a is located at distal end or distal end wall 34 of the implant.

For some applications, and as shown in Figs. 2A-B, movement of the anchor channel (e.g., by steering of catheter 22) directs first portion 70a towards first anchoring site 68a. For some applications, distal portion 62 comprising at least first portion 70a (e.g. distal end wall 34) of wall 28 is advanced out of distal opening 26 of catheter 22.

For some applications in which implant 20 comprises adjustment mechanism 40, and as shown in Fig. 2A, the adjustment mechanism is disposed distal to (i.e., in front of) the implant during advancement of the implant. For example, adjustment mechanism 40 can be disposed on the implant axis or axis d12 (e.g., collinearly with sleeve 30). For some such applications, mechanism 40 is coupled to sleeve 30 in a manner that allows mechanism 40 to move (e.g., to translate) from a state in which it is in line with axis d12, to a state in which it is disposed alongside sleeve 30 (Fig. 2B).

For some applications, one or more couplings 66 (e.g. sutures, etc.) facilitate translation of adjustment mechanism 40 by flexibly and/or articulatably coupling the mechanism to sleeve 30. For some applications, guide member 46 is tensioned or relaxed in order to reposition mechanism 40.

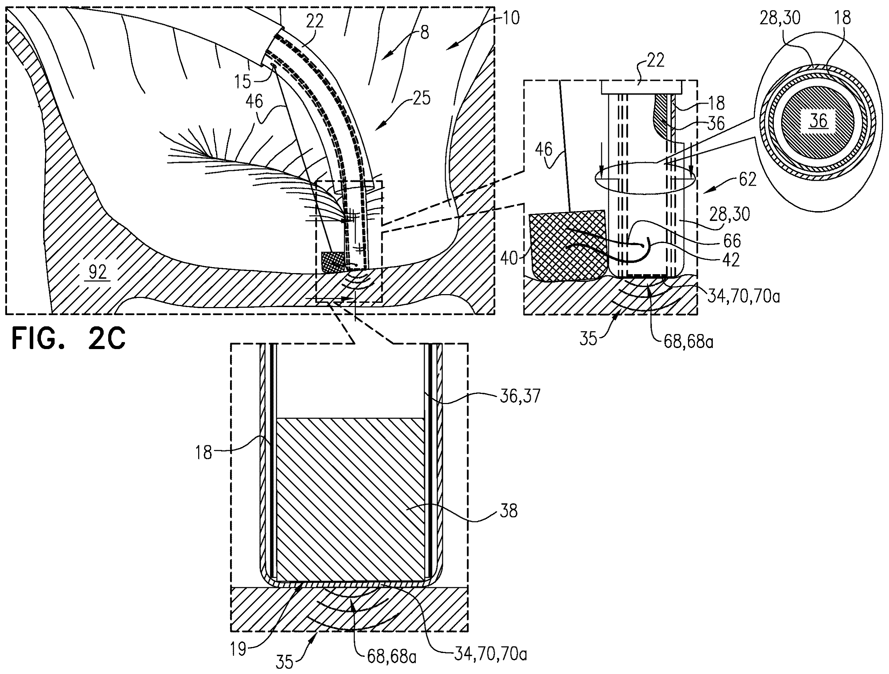

For some applications it is advantageous to (1) advance implant 20 to the tissue while mechanism 40 is disposed on axis d12 (e.g., collinearly with the sleeve), so as to maintain a small cross-sectional diameter of the implant for transluminal delivery; and (2) to subsequently move mechanism 40 away from the implant axis, e.g., so as to allow end wall 34 of the sleeve to be placed against an atrial surface of annulus 88 (Fig. 2C).

For some applications, anchor channel 18 exerts a pushing force upon a distal end or other portion of the implant. For some applications, anchor channel 18 exerts a pushing force upon distal end wall 34 from within sleeve 30. For some such applications, the pushing force exerted by channel 18 upon end wall 34 facilitates advancing first portion 70a out of distal opening 26 of catheter 22. In this way, first portion 70a (e.g. distal end wall 34) of the wall is positioned against first site 68a of cardiac tissue 92 such that: the distal region 24 of anchor channel 18 is disposed within lumen 44, and distal aperture 19 of anchor channel 18 is disposed at first portion 70a (Fig. 2C).

As shown, first portion 70a (e.g. distal end wall 34) of the implant can be placed against first site 68a disposed: (i) distally from the portion, and (ii) opposite distal opening 26 of catheter (Fig. 2C). For some such applications, the pushing force exerted by channel 18 upon first portion 70a facilitates holding portion 70a against first site 68a of the tissue, e.g., sandwiching portion 70a between tissue 92 and aperture 19, e.g., thereby defining site 68a as the site against which portion 70a is held by channel 18.

As shown in Fig. 2C, delivery tool 8 further comprises an ultrasound tool 36 which facilitates imaging of first site 68a by transmitting ultrasound energy to the site. For some applications, ultrasound tool 36 facilitates imaging of first site 68a by transmitting ultrasound energy through first portion 70a of the implant (e.g., through the wall) and into the site. Typically, and as shown, ultrasound tool 36 has an ultrasound transceiver 38 at a distal end 37 thereof.

For some applications, where the implant includes a wall through which an anchor passes to secure the wall to tissue (e.g., a side wall, a wall circumscribing or defining a lumen, etc.), positioning the ultrasound tool and/or a transceiver thereof such that the wall is held between the ultrasound too and/or transceiver and the tissue or site of the tissue (e.g., when the ultrasound tool is inside the lumen of an implant, etc.), holding the wall of the implant against a tissue site, and imaging the site can beneficially help optimize positioning and anchoring of the implant to the tissue (e.g., to an annulus of a native heart valve) while avoiding obstacles, blood vessels, etc. This can help ensure that the wall is held in an ideal or optimized location(s) for when an anchor is driven through the wall into the tissue to secure the implant to the tissue and, similarly, for subsequent sites and anchoring actions. Where the ultrasound tool is positioned inside a lumen of an implant, additional benefits (e.g., less traumatic interaction with tissue, protecting the transceiver, etc.) may come from having the ultrasound tool enclosed within the lumen during imaging.

Ultrasound tool 36 can be advanced distally, within longitudinal cavity 50 of anchor channel 18, to a desired position (e.g., at or near a site of the tissue). For example, in some applications, ultrasound tool 36 can be advanced distally, within longitudinal cavity 50 of anchor channel 18, to a position that is: within lumen 44 of implant 20 and facing first portion 70a of the wall (Fig. 2C). For some applications, ultrasound tool 36 is advanced distally through longitudinal cavity 50 while distal aperture 19 is disposed at the tissue site and/or at first portion 70a, e.g., such that ultrasound transceiver 38 is positioned such that it can transmit ultrasound energy through aperture 19 of channel 18. For some applications, ultrasound tool 36 is advanced distally such that a distal end of the ultrasound tool is generally flush with distal aperture 19. For some applications, ultrasound tool 36 is advanced distally such that at least a portion of ultrasound transceiver 38 protrudes distally through distal aperture 19, as shown in Fig. 2C.

Ultrasound tool 36 is used to image first site 68a by transmitting ultrasound energy 35 into first site 68a. For some applications, ultrasound tool 36 is used to image first site 68a by transmitting ultrasound energy 35 through first portion 70a and into first site 68a. That is, the ultrasound energy reaches first site 68a by passing through first portion 70a of wall 28.

While ultrasound transceiver 38 is within cavity 50 of anchor channel 18, a portion of the transmitted ultrasound energy is reflected from site 68a back to the transceiver, which detects the reflected ultrasound energy. For some applications, while ultrasound transceiver 38 is within cavity 50 of anchor channel 18 and facing first portion 70a, a portion of the transmitted ultrasound energy is reflected from site 68a back to the transceiver (via portion 70a of wall 28), which detects the reflected ultrasound energy.

Ultrasound tool 36 may therefore be considered to be "forward-looking," in that the ultrasound tool can be used to image first site 68a while: (i) the site is disposed distally from the implant and opposite distal opening 26 of catheter 22, (ii) ultrasound transceiver 38 is in a position that is facing the first site 68a (which can be, for example, a position that is within lumen 44 of the implant and facing first portion 70a of the wall), and (iii) ultrasound energy is both transmitted by the ultrasound transceiver, and reflected back to the ultrasound transceiver (e.g., through first portion 70a).

For some applications, anchor channel 18 (e.g. distal aperture 19 thereof) is used to hold first portion 70a against first site 68a. For some such applications, ultrasound tool 36 is advanced distally through longitudinal cavity 50, while aperture 19 is holding first portion 70a against first site 68a.

It is hypothesized that holding first portion 70a against first site 68a by anchor channel 18, and/or extending distal end 37 of ultrasound tool 36 through distal aperture 19 of anchor channel 18, facilitates use of ultrasound tool 36 to image the site, as described herein below.

Typically, the reflected ultrasound energy is translated, using ultrasound tool 36, into data indicative of the reflected ultrasound energy (hereafter "data") detected by ultrasound transceiver 38. For some applications, and as shown in Fig. 2C, ultrasound tool 36 further comprises an ultrasound controller 74, electronically connected (e.g., via one or more connectors 72) to transceiver 38, and configured to facilitate analysis of the reflected ultrasound energy detected by the ultrasound transceiver.

Ultrasound controller 74 can comprise circuitry 75, which is used to assess and/or analyze the ultrasound data. For example, ultrasound controller 74 can be used to operate a data analysis program in circuitry 75. Alternatively or in addition, an image reflective of the data can be displayed on a user interface 60 (e.g., monitor, screen, etc.) for an operator to evaluate.

Analysis of the ultrasound data may provide a range of analysis output parameters. For some applications, the analysis parameters can be displayed on ultrasound controller 74 (e.g. on user interface 60 thereof). For example, analysis of the data may provide, inter alia, a depth of tissue into which ultrasound energy is transmitted. For some such applications, a tissue depth of at least 7 mm (e.g., 7-20 mm) may indicate, alone or in conjunction with other parameters, that site 68 is suitable for anchoring tissue anchor 52. Alternatively or in addition, analysis of the data may provide a radiodensity of tissue into which ultrasound energy is transmitted. For some such applications, a radiodensity of at least 60 Hounsfield units (HU) and/or less than 600 HU (e.g., 60-600 HU) may indicate, alone or in conjunction with other parameters, that site 68 is suitable for anchoring tissue anchor 52. For some applications, setting an upper radiodensity limit (such as 600 HU) may be used to facilitate identification and avoidance of calcified tissue.

Analysis of the ultrasound data may provide additional information which can be displayed to the operator as a warning message on ultrasound controller 74 (e.g. on user interface 60 thereof). For example, analysis of the ultrasound data may indicate that: (i) ultrasound transceiver 38 is not in sufficient proximity to tissue 92 (e.g., is not pressing on the tissue), (ii) a blood vessel 58 is present at the site of the tissue, and/or (iii) another part of implant 20 (e.g. contraction member 42) is disposed between portion 70 of the implant and the site. Ultrasound controller 74 can be configured to display additional warning messages, as relevant to the particular implant 20 and tissue 92 with which system 10 is used.

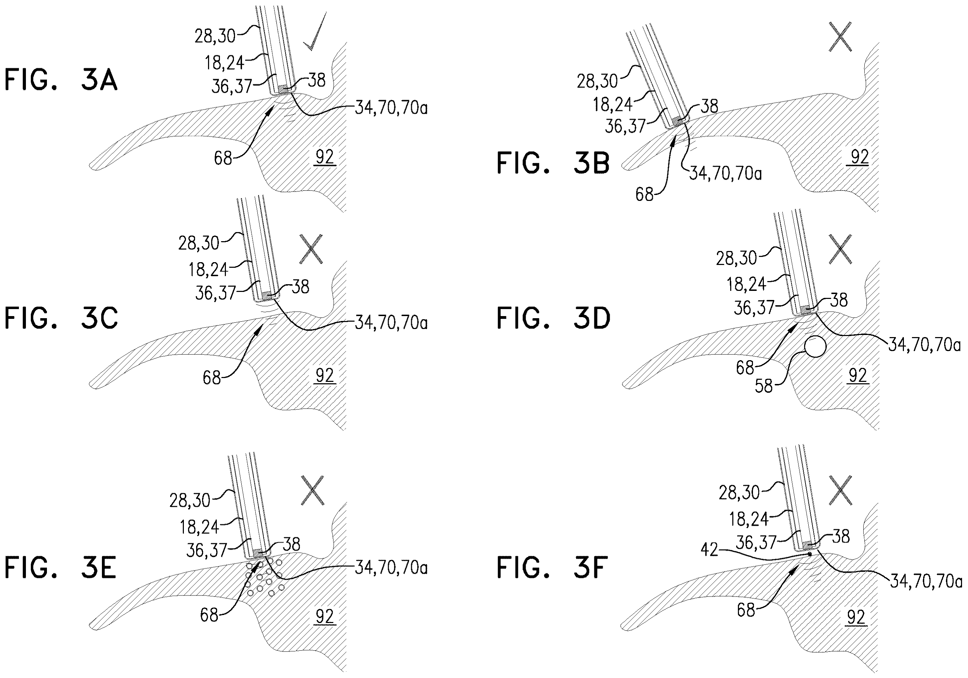

Reference is also made to Figs. 3A-F, which are schematic illustrations showing various positions of ultrasound tool 36 with respect to cardiac tissue 92, in accordance with some applications of the invention. As shown, ultrasound transceiver 38 is disposed in distal region 24 of anchor channel 18, such that distal end 37 of ultrasound tool 36 extends to distal aperture 19 of the anchor channel. In the example shown, tool 36 is disposed such that distal end 37 (e.g. a portion of ultrasound transceiver 38) protrudes through distal aperture 19 (e.g., to first portion 70a). In this way, ultrasound energy 35 is transmitted from transceiver 38 (which can be through first portion 70a of the implant) and a portion of the ultrasound energy is reflected back to the transceiver (which can also be via portion 70a of the implant). The reflected energy that is detected by transceiver 38 is used (e.g., by the operator, e.g., facilitated by controller 74) to determine the suitability of the particular site 68 for receiving a tissue anchor 52 in order to anchor the implant to the tissue at the tissue site.

For some applications, and as shown in Fig. 3A, anchor channel 18 (e.g. distal aperture 19 thereof) is used to hold first portion 70a against site 68 of tissue 92, sandwiching sleeve 30 against cardiac tissue 92. Ultrasound energy 35 penetrates the tissue, and a portion of the energy is reflected back to transceiver 38. The check mark in Fig. 3A represents that the ultrasound data indicates that the particular site 68 of Fig. 3A is suitable for anchoring sleeve 30 to the tissue.

Figs. 3B-F represent various tissue sites 68 that may be unsuitable for anchoring sleeve 30 to the tissue, this unsuitability being determined using ultrasound tool 36. In each of these figures, this unsuitability is represented by an "X."

Figs. 3B and 3E show portion 70 of sleeve 30 having been placed against a site 68 at which tissue 92 is of less-than-optimal thickness (e.g. at a leaflet of a cardiac valve, Fig. 3B) or density (Fig. 3E). It is often undesirable to anchor implant 20 at such tissue sites, e.g., because they may lack sufficient strength to retain tissue anchor 52.

Figs. 3C shows first portion 70a of the wall having not been successfully placed in direct contact with site 68. It is generally preferable for portion 70a to be in contact with site 68, to facilitate placement of the ultrasound transceiver as close as possible to the site, e.g., sandwiching the wall of the implant between the transceiver and the site. Such sub-optimal positioning would be detectable using the ultrasound tool, and in such a situation the apparatus and/or implant can be repositioned. For some applications (e.g., for some applications in which system 310, described hereinbelow, is used), identifying that such optimal placement of the ultrasound transceiver has been achieved, may facilitate ensuring that the wall of the implant is held against the site of the tissue during anchoring. It is hypothesized that ensuring that the wall of the implant is held against the site of the tissue during anchoring facilitates optimal anchoring of tissue anchor 52 to tissue 92.

Fig. 3D shows portion 70 of sleeve 30 having been placed against a site 68 at which lies blood vessel 58. It may be undesirable to anchor implant 20 at such a tissue site. For example, driving tissue anchor 52 into such a site might result in damage to the blood vessel, which may increase a risk of surgical complications. For some applications, ultrasound controller 74 is configured to indicate, to the operator, the presence of blood vessel 58.

Fig. 3F shows portion 70 of sleeve 30 sandwiching another part of implant 20 (such as contraction member 42, as shown) between portion 70 and site 68. It may be undesirable to anchor implant 20 in such a situation. For example, the presence of the other part of implant 20 may interfere with anchoring, and/or functioning of the implant. For example, for some applications, anchoring implant 20 in such a situation might theoretically interfere with functionality of the contraction member.

As described herein above in reference to Figs. 3B-F, in the case that analysis of the data (e.g. by circuitry 75 of ultrasound tool 36, or by the operator) indicates that site 68 is unsuitable for anchoring the implant to the tissue, control assembly 64 can be used to steer distal part 25 of catheter 22 to an alternate site 68 of tissue 92. While anchor channel is used to position the implant at the alternate site 68, ultrasound tool 36 is used to image the alternate site 68, as described herein above in reference to Fig. 2C. For some applications, ultrasound tool 36 can be used to image a plurality of alternate sites 68, until analysis of the data indicates that the site is suitable for anchoring the implant and/or tissue anchor to tissue 92.

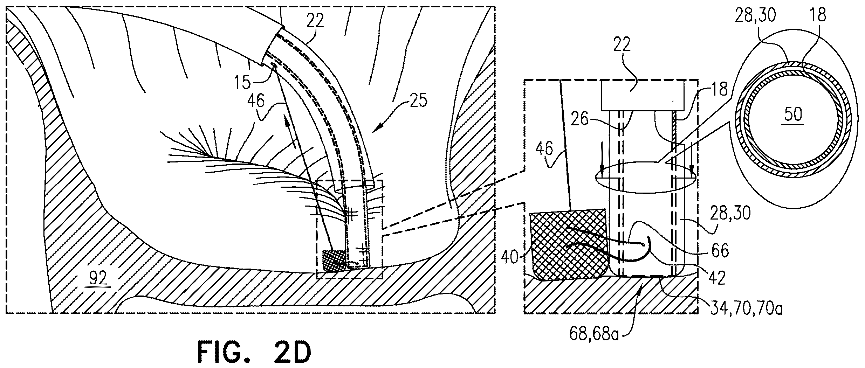

As described herein above in reference to Fig. 3A, if the data indicates that site 68 is suitable for anchoring the implant to the tissue, ultrasound transceiver 38 is withdrawn from longitudinal cavity 50 (for some applications, the ultrasound transceiver is withdrawn from lumen 44 of implant 20), through the proximal opening of the catheter. In some applications, this is done while holding (e.g., pressing) first portion 70a of wall 28 against site 68 with anchor channel 18 (e.g. distal aperture 19 thereof), e.g., as shown in Fig. 2D, mutatis mutandis.

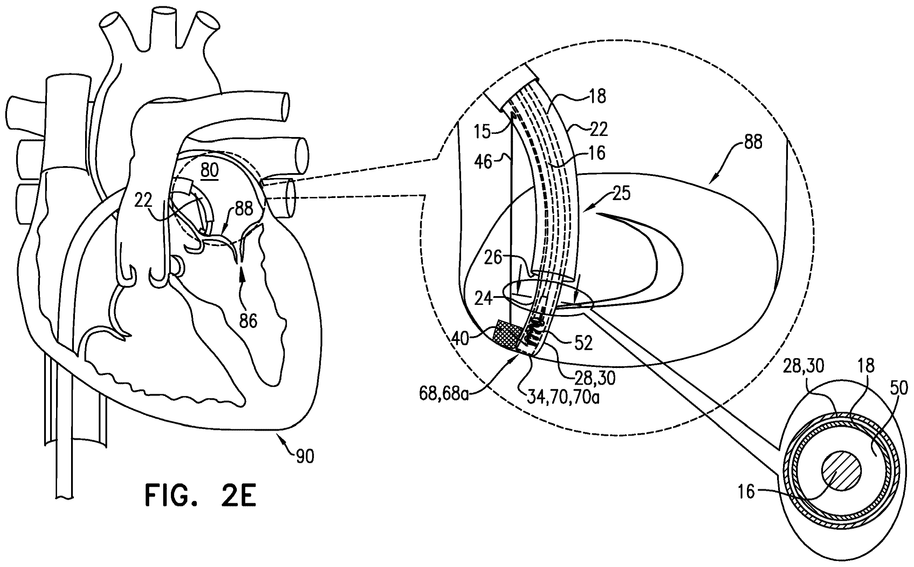

As shown in Fig. 2E, tool 8 further comprises an anchor driver 16 configured to anchor the implant to the tissue by driving tissue anchor 52 (e.g., a tissue-penetrating portion thereof) into first site 68a. Where the implant includes a sleeve or other lumen, the anchor 52 can be driven through first portion 70a of implant 20 (e.g., sleeve 30 thereof) and then into the first site 68a. Anchor driver 16 can comprise an elongate and flexible shaft (which can be tubular or another design). For some applications, driver 16 could be as described, mutatis mutandis, in US Patent Application Publication 2018/0049875 to Iflah et al., which is incorporated by reference herein.

For some applications, system 10 and/or implant 20 comprises a plurality of tissue anchors 52, often between about 5 and about 20 anchors, such as about 10 or about 16 anchors. Often, but not necessarily, anchors 52 comprise a biocompatible material such as stainless steel 316 LVM. For some applications, anchors 52 comprise nitinol. For some applications, anchors 52 are coated fully or partially with a non-conductive material.

For some applications, and as shown in Figs. 2E-F, driver 16 is advanceable through longitudinal cavity 50 to implant 20 only while the ultrasound transceiver is not disposed through the cavity. That is, for such applications, at a given time either ultrasound transceiver 38 or anchor driver 16 can be disposed through cavity 50 of anchor channel 18.

Driver 16 can be advanced, while coupled to tissue anchor 52, through longitudinal cavity 50 of channel 18, to the implant. For some applications, driver 16 advances anchor 52, while coupled to driver 16, into lumen 44 of the implant, in a manner in which the anchor reaches first portion 70a of the implant or wall, which, as described hereinabove, is opposite aperture 19.

As described hereinabove, first portion 70a can be defined by virtue of the position of channel 18 within lumen 44, e.g., first portion 70a can be defined as being opposite aperture 19. First site 68a is the site of the tissue to which implant 20 (e.g., first portion 70a thereof) is intended to be anchored and can be disposed distally from first portion 70a. First site 68a can be opposite distal opening 26 of catheter 22. Therefore, first portion 70a can be the portion of the implant (e.g., the portion of wall 28 thereof) that is disposed between aperture 19 and first site 68a.

For some applications, driver 16 advances anchor 52 through anchor channel 18 and to first portion 70a, while first portion 70a is already disposed at first site 68a. For some applications, and as shown, anchor channel 18 (e.g. distal aperture 19 thereof) is used to hold first portion 70a against first site 68a, sandwiching first portion 70a between the aperture and first site 68a, as: (i) anchor driver 16 is advanced through cavity 50 to implant 20 (Fig. 2E), and (ii) first portion 70a of wall 28 is anchored to first site 68a.

It is hypothesized that holding first portion 70a of wall 28 against first site 68a with anchor channel 18 throughout (i) imaging of site 68, (ii) withdrawal of ultrasound transceiver 38 from longitudinal cavity 50, (iii) advancement of anchor driver 16 distally through longitudinal cavity 50 to implant 20, and (iv) anchoring of first portion 70a of wall 28 to first site 68a of the tissue, contributes to accuracy with which the implant is anchored to the tissue. That is, holding first portion 70a of wall 28 steadily against first site 68a using anchor channel 18 facilitates anchoring implant 20 at the particular suitable site 68 that was identified using ultrasound tool 36.

Heretofore, tissue anchor 52 has been described as first tissue anchor 52a, portion 70 has been described as first portion 70a, and site 68 has been described as first site 68a. Accordingly, anchoring implant 20 can comprise driving first anchor 52a through first portion 70a of wall 28 and into first site 68a of tissue 92.

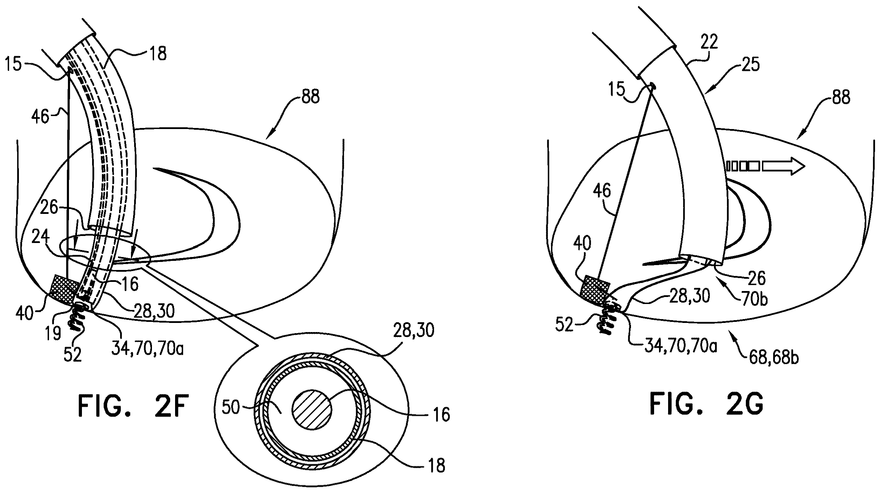

For some applications, and as shown in Figs. 2G-K, anchoring implant 20 can comprise anchoring one or more additional portions 70 of wall 28 to additional sites of tissue 92 (e.g. of annulus 88), by driving additional tissue anchors 52 through the respective portions of the wall and into the respective sites. Similar to the description herein above in reference to first portion 70a and first site 68a, each additional portion 70 can be held against the respective site 68, the site is imaged using ultrasound tool 36, and the anchor 52 is used to anchor the portion to the site 68. Often, and as described herein above in reference to Figs. 3A-F, data indicative of reflected ultrasound energy is analyzed to determine suitability of each site 68 prior to anchoring anchor 52.

Fig. 2G shows anchor driver 16 having been withdrawn, and a second portion 70b of sleeve 30 (i.e., a portion of the sleeve that is proximal to end wall 34) having been released from channel 18 by retracting channel 18 proximally with respect to the sleeve. Depending on the tension applied between the first and second tissue anchor sites, the section of sleeve 30 disposed between first portion 70a and second portion 70b may remain tubular in shape or may become flattened.

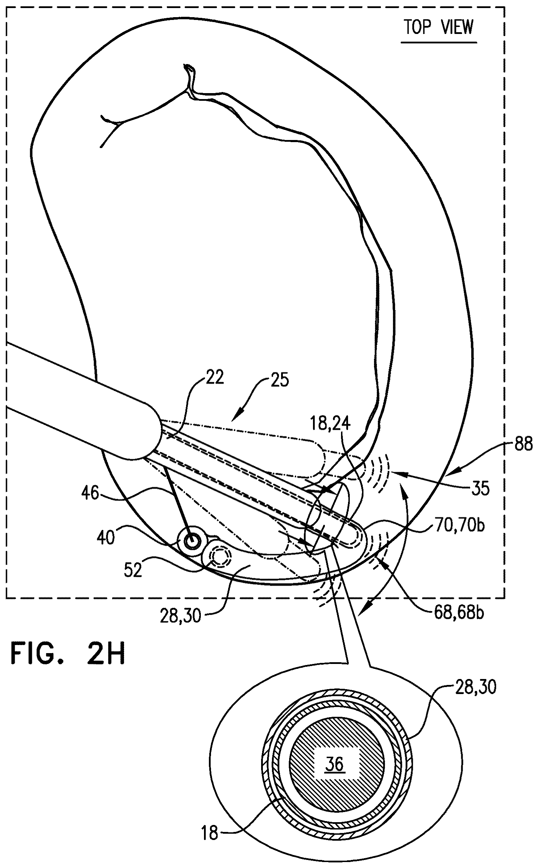

As shown in Fig. 2H, second portion 70b can be positioned at a second anchoring site 68b, e.g., by repositioning distal part 25 of catheter 22, and ultrasound tool 36 is re-advanced distally to distal region 24 of anchor channel 18. The re-advancement of ultrasound tool 36 can be performed before or after the positioning of portion 70b at site 68b. As described hereinabove, anchor channel 18, anchor driver 16 and ultrasound tool 36 can be dimensioned such that only one of the anchor driver or the ultrasound tool can be disposed within longitudinal cavity 50 of anchor channel 18 at a given time. Because anchor driver 16 has been withdrawn from cavity 50, ultrasound transceiver 38 can be advanced distally through the cavity to the second anchoring site and/or to implant 20 (e.g. facing second portion 70b).

Fig. 2H illustrates that distal part 25 of catheter 22 can be repositioned multiple times to multiple potential tissue sites, until a suitable site 68b is identified.

For some applications, a maximum distance between first site 68a and second site 68b is provided by the length of sleeve 30 that was previously released from channel 18. That is, for some applications, distal part 25 of catheter 22 can be repositioned to second site 68b located anywhere within an arc having a radius that equals the maximum distance, centered on first tissue anchor 52a.

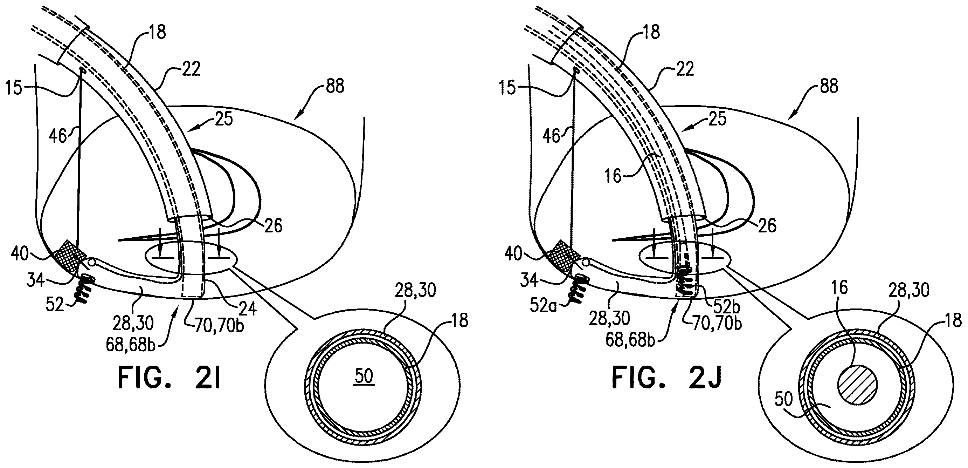

As described herein above in reference to Fig. 2D, Fig. 2I shows ultrasound tool 36 having been retracted from anchor channel 18. As described for first anchor 52a, mutatis mutandis, anchor channel 18 (e.g. distal aperture 19 thereof) can be used to hold second portion 70b against second site 68b while ultrasound tool 36 is withdrawn, and anchor driver 16 is re-advanced with a second anchor 52b.

Further similarly to as anchoring of first anchor 52a, Fig. 2J shows anchor driver 16 having been advanced distally within cavity 50 of anchor channel 18, to second portion 70b. As described above regarding anchoring of first anchor 52a, Figs. 2J-K show an example where second tissue anchor 52b being deployed through second portion 70b of wall 28. Second tissue anchor 52b can be deployed by driving the second anchor to penetrate and pass through wall 28 of sleeve 30 into cardiac tissue 92 at second site 68b.

As shown, second anchor 52b can be deployed proximally (with respect to implant 20) from first anchor 52a.

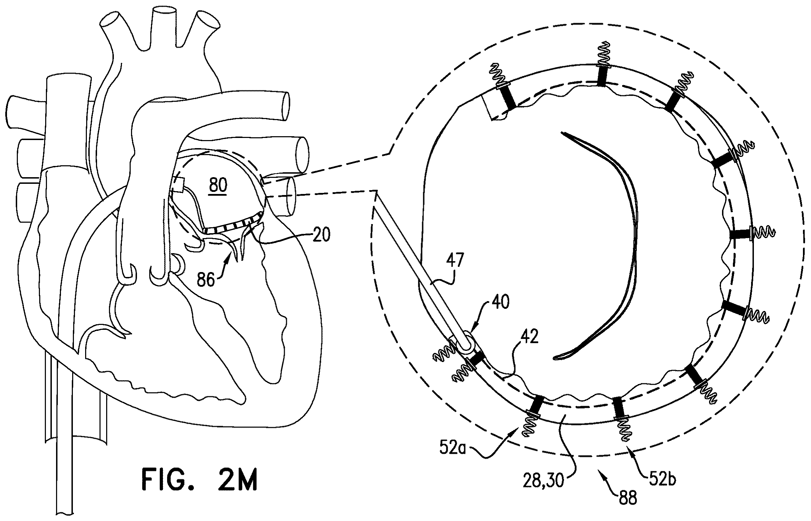

Fig. 2M shows the entire length of implant 20 (e.g., sleeve 30) having been anchored, via a plurality of anchors 52, to annulus 88. For some applications, and as shown, implant 20 (e.g., sleeve 30) is anchored around the annulus between the right fibrous trigone and the left fibrous trigone. After anchoring is complete, channel 18 is withdrawn. For some applications, catheter 22 is also withdrawn.

After implant 20 is anchored to annulus 88, implant 20 (e.g., sleeve 30 or the contracting portion thereof) can be contracted, e.g., by tensioning contraction member 42. As described hereinabove, adjustment mechanism 40 is configured to adjust a length of implant 20 (e.g. sleeve 30 of the annuloplasty structure) by tensioning contraction member 42, e.g., as described in US Patent Application Publication 2018/0049475 to Iflah et al., mutatis mutandis.

For such applications, guide member 46 can remain coupled to implant 20 (e.g., to adjustment mechanism 40) after the removal of channel 18 (and optionally also of catheter 22). As shown in Fig. 2M, an adjustment tool 47 can be advanced along (e.g., over and along) guide member 46 to adjustment mechanism 40 and can be used to actuate the adjustment mechanism. Adjustment tool 47 can comprise a rotation tool, and can be configured to actuate (e.g., rotate) adjustment mechanism 40, so as to tension contraction member 42, and thereby contract implant 20 (e.g., sleeve 30, etc.), as described hereinabove. For some applications, adjustment mechanism 40 comprises a rotatable spool, to which contraction member 42 is coupled, rotation of the spool reversibly adjusting a degree of tension of contraction member 42. Due to the anchoring of portions 70 to anchoring sites 68, adjusting (e.g., reducing) the length of the structure adjusts (e.g., reduces) the perimeter of annulus 88.

For some applications, the implant 20 does not comprise a discrete adjustment mechanism, and instead the contraction member is tensioned by pulling proximally, e.g., using an adjustment tool or tools, e.g., while providing a reference force against the implant. For such applications, a locking member or lock (e.g., clip, fastener, staple, crimper, clamp, etc.) can be locked to contraction member 42 in order to lock in the applied tension. For some such applications, the locking member or lock is a component of the implant and is therefore introduced into the subject simultaneously with the rest of the implant. Optionally, the locking member or lock can be introduced subsequent to the introduction of the implant into the subject, e.g., with the adjustment tool. Implants without a discrete adjustment mechanism as described here can include a sleeve and/or a lumen, or can be sleeveless or without a lumen (e.g., some can comprise a contraction member and tissue anchors without a sleeve or fabric, etc.)

Once the desired level of adjustment of structure is detected, e.g., by monitoring the extent of regurgitation of the valve using echocardiography (such as Doppler echocardiography) and/or fluoroscopy, adjustment tool 47 and guide member 46 are withdrawn from the heart. Typically, implant 20 is detached from delivery tool 8, and the delivery tool is transluminally retracted from heart 90 of the subject.

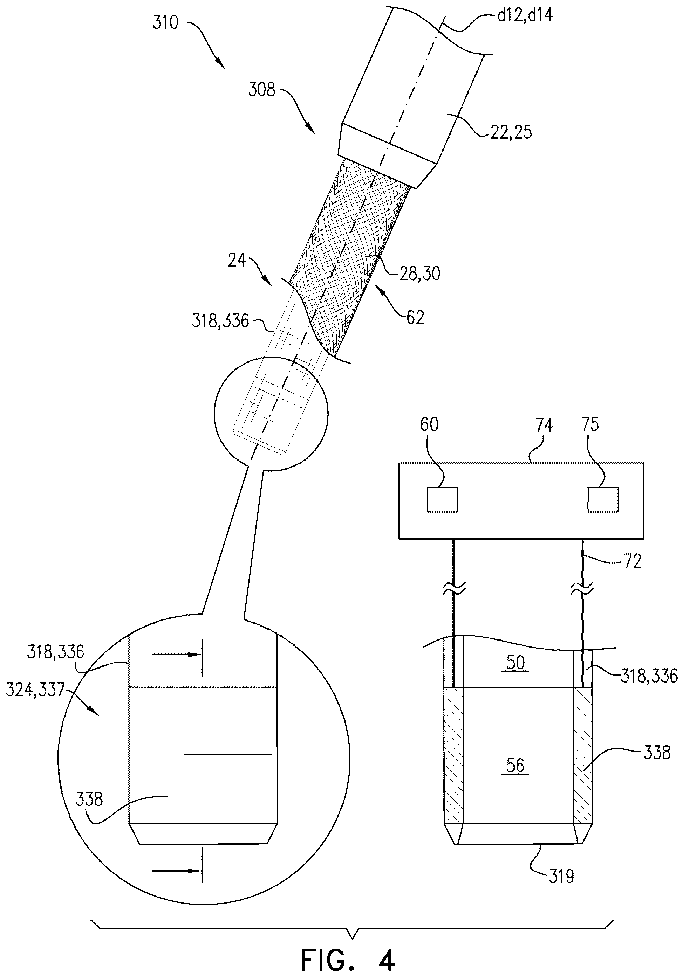

Reference is made to Fig. 4, which is a schematic illustration of a multi-component system 310 comprising implant 20, and a delivery tool 308 for delivering the implant to a tissue of a subject, in accordance with some applications.

Except where noted, system 310 can be identical or similar to system 10 described hereinabove and can be used in a similar way to system 10, mutatis mutandis. For example, components that are identically named between the systems generally share similar features and serve similar functions as each other. Components bearing identical reference numerals are generally interchangeable between delivery tools 8 and 308. As such, the description below of system 310 focuses upon features that are particular to system 310.

Delivery tool 308 includes an ultrasound tool 336 that comprises an ultrasound transceiver 338 and an anchor channel 318. That is, whereas the ultrasound tool of system 10 is discrete from, and movable with respect with, the anchor channel of system 10, the ultrasound tool of system 310 is a component of the anchor channel of system 310. For such applications, ultrasound transceiver 338 can be fixedly positioned at a distal end 337 of ultrasound tool 336 (e.g., at a distal end of channel 318). Often for such applications, ultrasound tool 336 is advanceable within catheter 22 and implant 20 in a manner similar to as described herein above in reference to anchor channel 318 of delivery tool 8. Further similarly to anchor channel 18 described above, ultrasound tool 336 (e.g., anchor channel 318 thereof) can be used to press portions of implant 20 and/or a sleeve 30 against tissue sites 68.

As shown in Fig. 4, ultrasound transceiver 338 is shaped as a ring defining a passage 56 therethrough (other shapes and configurations are also possible). A distal aperture 319 of ultrasound tool 336 can be defined by channel 318, but may alternatively or additionally be defined by transceiver 338. Passage 56 can be continuous with longitudinal cavity 50. For some applications, passage 56 has an internal diameter that is no less than 80 percent (e.g., no less than 90 percent) as great as an internal diameter of cavity 50. For example, the internal diameter of passage 56 can be about the same size as the internal diameter of cavity 50.

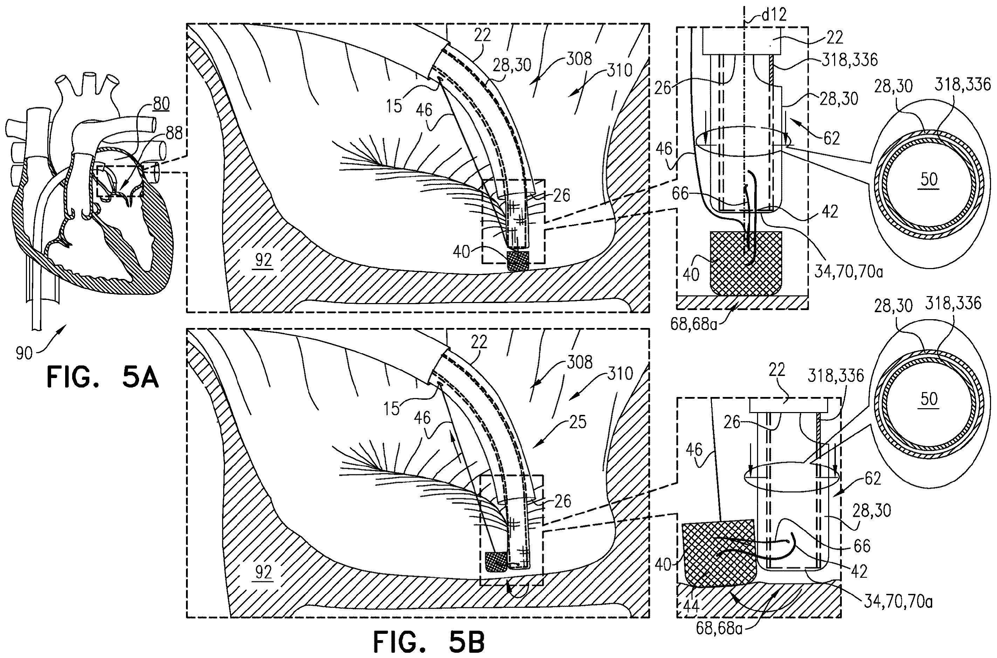

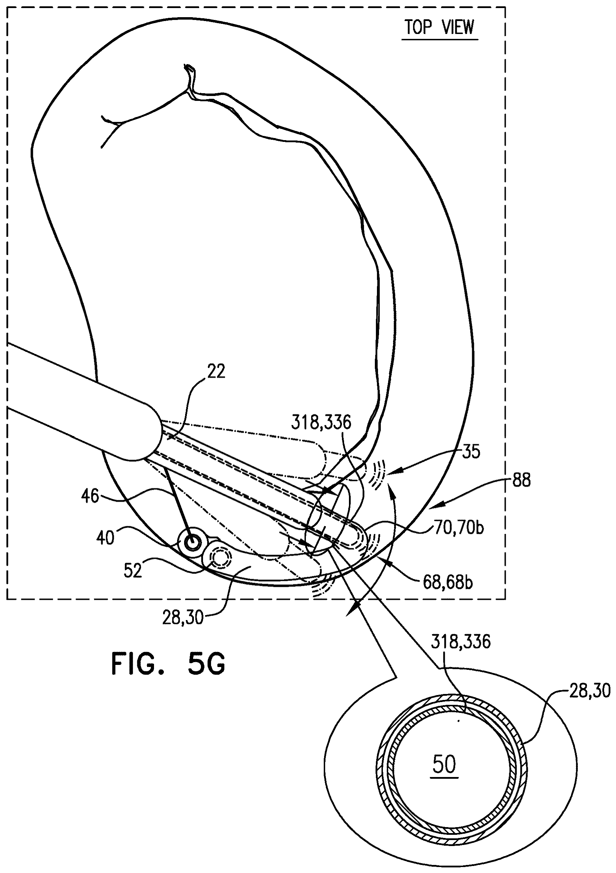

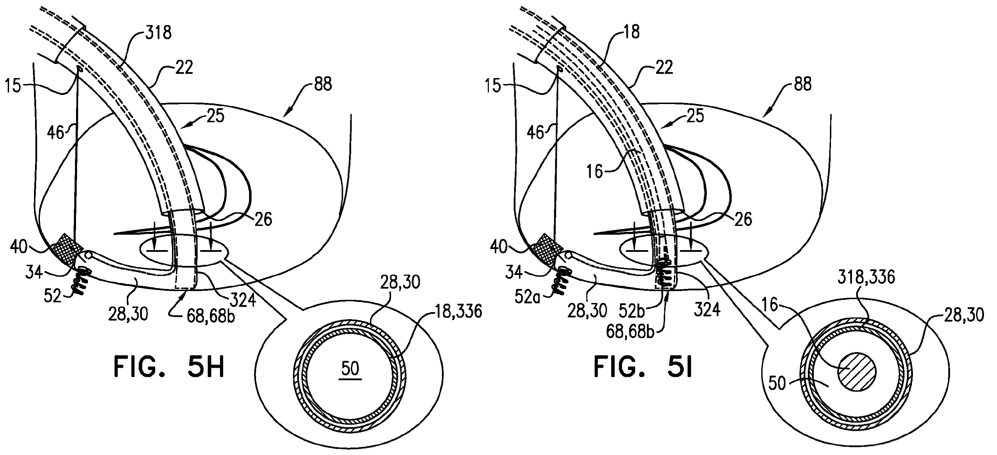

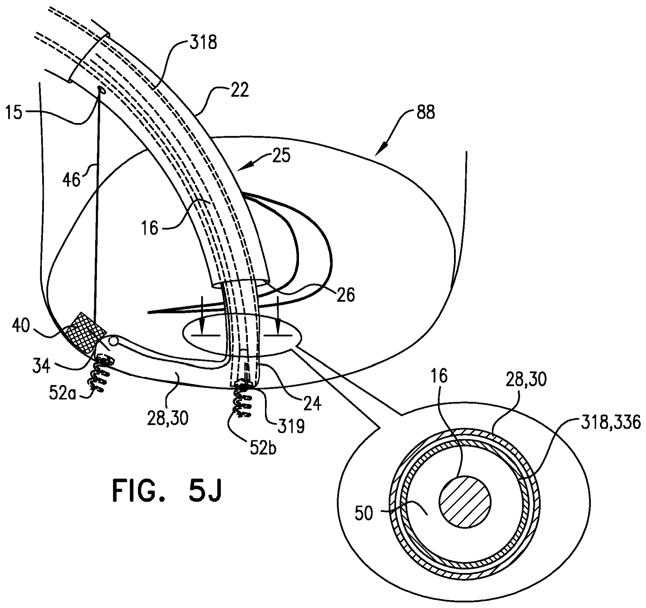

Reference is made to Figs. 5A-J, which are schematic illustrations showing tool 308 being used to deploy implant 20 at cardiac tissue 92 of heart 90, in accordance with some applications of the invention.

As shown in Fig. 5A-B, ultrasound tool 336 can be positioned with ultrasound transceiver 338 within a lumen 44 of an implant and facing first portion 70a of the wall. As shown, movement of adjustment mechanism 40 from a state in which it is in line with sleeve axis d12 (Fig. 5A), to a state in which it is disposed alongside sleeve 30 (Fig. 5B), can be accomplished similarly to as described for tool 8, mutatis mutandis.

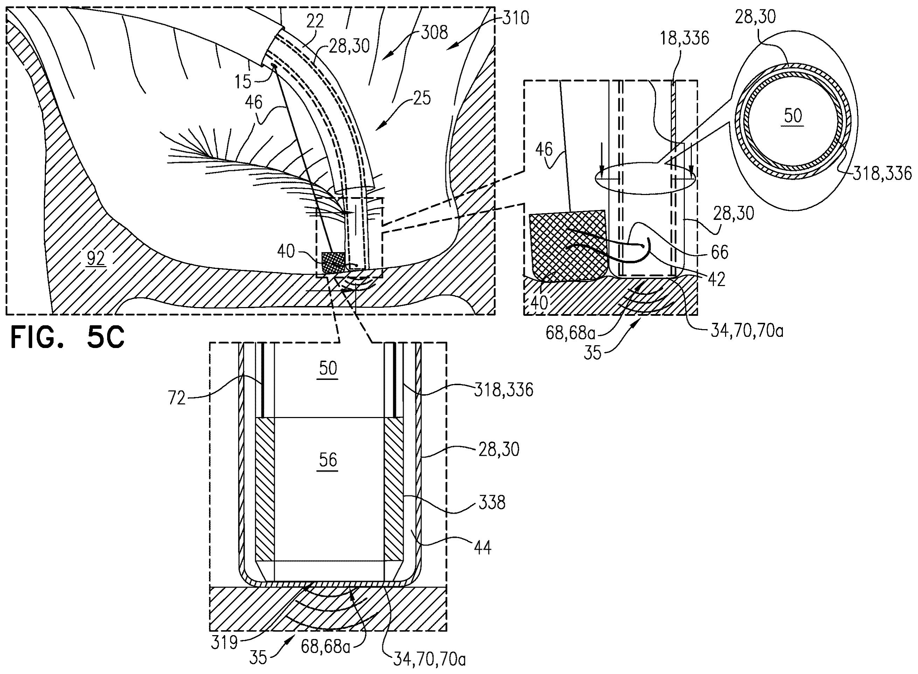

As shown in Fig. 5C, ultrasound tool 336 (e.g., channel 318 thereof) can be used to sandwich portion 70a of implant 20 (e.g., of wall 28) against site 68a of tissue 92 and transmit ultrasound energy 35 into the site (e.g., through the portion). Ultrasound tool 336 may be considered to be "forward-facing" in the same way as ultrasound tool 36 of delivery tool 8, regardless of ultrasound tool 336 being shaped as a ring. For some applications, ultrasound transceiver 338 defines distal aperture 319, which can be used to press portion 70 against site 68 (Fig. 5C).

Notable in the transition between Figs. 5B and 5C is the absence of a step in which an ultrasound tool is advanced within an anchor channel, since, in delivery tool 308, the ultrasound tool itself comprises the anchor channel.

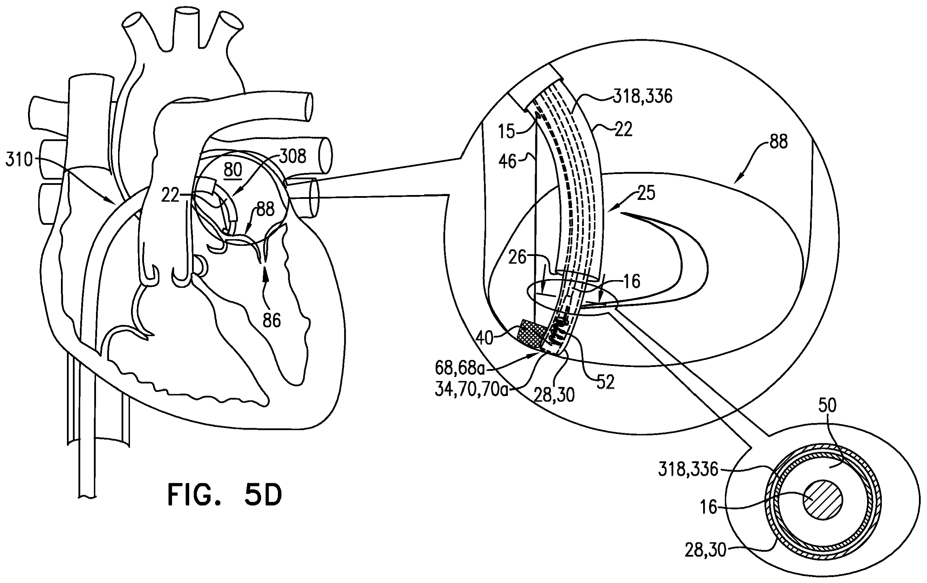

Fig. 5D shows anchor driver 16 having been advanced, while coupled to tissue anchor 52, through longitudinal cavity 50 and passage 56, such that tissue anchor 52 reaches first portion 70a.

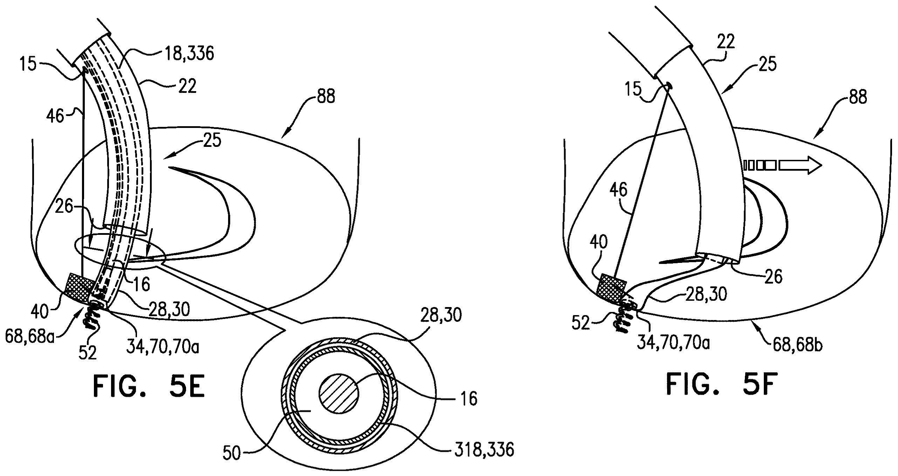

Notable also, in the transition between Figs. 5C and 5D, is the absence of a step in which ultrasound tool 336 is withdrawn, prior to advancing anchor driver 16 toward portion 70a. Since ultrasound tool 336 comprises anchor channel 318, and passage 56 is a distal part of cavity 50, anchor driver 16 can be advanced through the cavity defined by the ultrasound tool, and prior removal of the ultrasound tool is not necessary. Tissue anchor 52 is driven through passage 56 (and optionally through a portion 70a) and into site 68a (Fig. 5E).

In contrast to delivery tool 8, in delivery tool 308, both ultrasound tool 336 and anchor driver 16 can be disposed at a location or site simultaneously. In one application, both ultrasound tool 336 and anchor driver 16 can be simultaneously disposed within lumen 44 of implant 20. Thus, delivery tool 308 obviates the need to alternate between advancing either ultrasound tool 336 or anchor driver 16. This arrangement can beneficially allow use of the ultrasound tool and anchor driver together or simultaneously, e.g., allowing the ultrasound tool to be used as the anchor driver attaches an anchor to tissue at the site to visualize entry of the anchor into the tissue and placement within the tissue, etc. The ultrasound tool and anchor driver can be configured to be coaxial with each other as used together (and, optionally, the tissue anchor can be coaxial with a distal region or end of the ultrasound tool and/or the transceiver), which may help provide improved visualization and/or attachment of the anchor to the tissue.

For some applications, and similarly to as described in reference to delivery tool 8 in Figs. 2G-K, delivery tool 308 can be used to anchor one or more additional portions 70 of wall 28 to additional sites of tissue 92. Fig. 5F shows delivery tool 308 being used to release a second portion 70b of sleeve 30 from ultrasound tool 336, after which distal part 25 of catheter is repositioned, so as to position second portion 70b at second anchoring site 68a (Figs. 5F-H). Often for delivery tool 308 and as shown, second site 68b of tissue 92 is assessed without removing ultrasound transceiver 38 from within lumen 44 of implant 20 (i.e. without removing the ultrasound transceiver between assessing first site 68a and assessing the second site).

Figs. 5I-J show second tissue anchor 52b being advanced, using anchor driver 16, through cavity 50 and passage 56 and driven through second portion 70b into second site 68b. Further, for delivery tool 308 and as shown, second tissue anchor 52b can be driven through second portion 70b of wall 28 and into second site 68b of tissue 92 while ultrasound transceiver 38 remains within lumen 44 of implant 20 and facing the second portion of the wall.

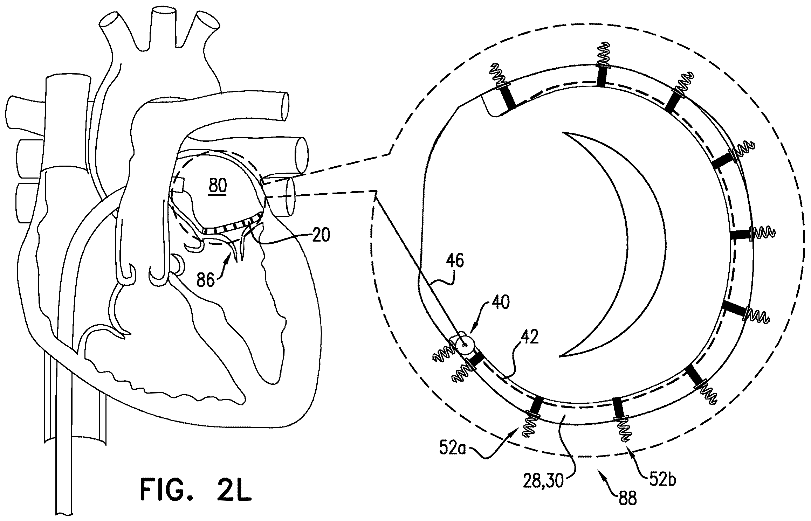

The manner in which an implant can be contracted, e.g., by tensioning contraction member 42, is generally not affected by the differences described herein below between delivery tools 8 and 308, and contraction can therefore be accomplished as described above in reference delivery tool 8 in Figs. 2L-M.

Although embodiments described and illustrated herein often relate to annuloplasty structures, such as annuloplasty bands, anchored to tissue of an annulus of a native heart valve, the methods, techniques, systems, apparatuses, etc. disclosed herein are relevant to anchoring a range of implants to various tissue of a subject, mutatis mutandis.

The apparatuses, systems, methods, techniques, etc. described herein can be used in combination with those described in US Patent Application Publication No. 2018/0049475 to Iflah et al., PCT Patent Application No. PCT/IL2019/050777 to Brauon et al. (which published as WO 2020/012481), US Provisional Patent Application No. 62/949,392 to Kasher et al., and/or US Patent No. 9,949,828 to Sheps et al, each of which is incorporated by reference herein for all purposes.

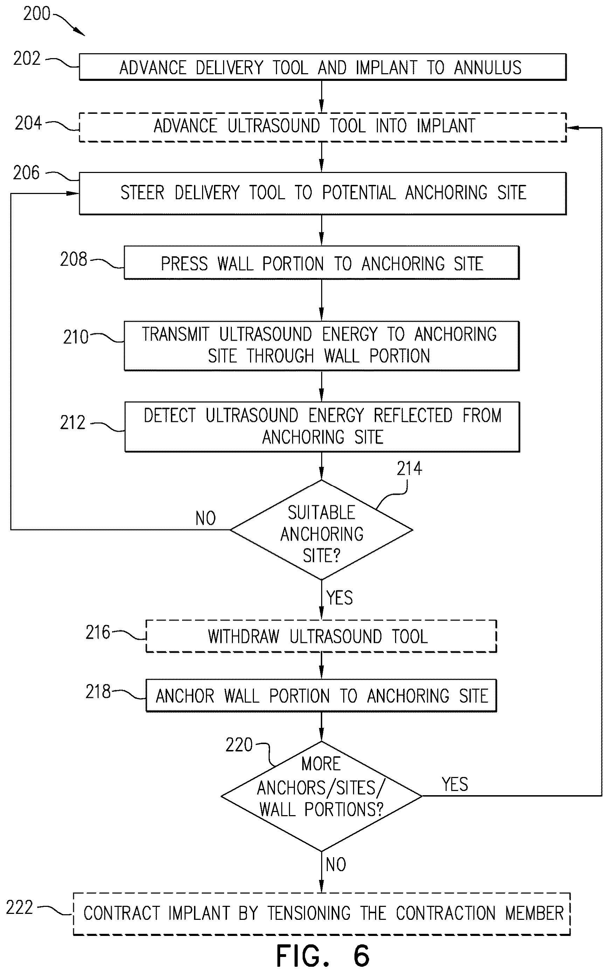

Reference is made to Fig. 6, which is a flow chart that schematically illustrates at least some steps of an example method 200 for implanting an implant at a tissue, in accordance with some applications. For some applications, method 200 is performed using delivery tool 8 and/or delivery tool 308. For some applications, method 200 is used to implant the implant at cardiac tissue 92 of heart 90 of a subject. For some applications, method 200 is performed on implant 20.

An implant having a wall that surrounds a lumen (e.g., implant 20) is advanced, often within a delivery tool such as delivery tool 8 or 308, to a tissue such as annulus 88 (step 202). For some applications, an ultrasound tool such as ultrasound tool 36 or 336 is then advanced into implant 20 (step 202). (Optionally, the ultrasound tool may be present within the implant during advancement of the implant). The delivery tool is steered to a potential anchoring site (step 206), and a portion of the implant (e.g., a portion of a wall or sleeve of the implant, etc.) can be pressed (e.g. held) against the site (step 208), e.g., as described with reference to Figs. 2C and 5C.

Ultrasound energy is transmitted (e.g. using ultrasound transceiver 38 or 338, respectively, of ultrasound tool 36 or 336), which can be done through the portion of the implant, to the site of the tissue (step 210). A portion of the ultrasound energy (e.g. ultrasound energy reflected from the site and/or through the portion of the wall) is detected, as described in reference to Fig. 2C (step 212).

The site of the tissue is then assessed (e.g. analyzed) for suitability as an anchoring site at which to anchor the implant (step 214). For example, the site can be assessed as described with reference to Figs. 2C and 3A-F. In the case that assessment of the site results in the site being found to be unsuitable as the anchoring site, the delivery tool is steered to another potential anchoring site (step 204).

In the case that assessment of the site results in the site being found to be suitable as the anchoring site, the portion is anchored to the site, e.g. using anchor driver 16 as described in reference to Figs. 2E-F (step 218). For some applications, the ultrasound tool is withdrawn from the delivery tool, prior to anchoring the portion of the wall to the site of the tissue, as described in reference to Fig. 2D. (Optionally, the portion is anchored to the site while the ultrasound transceiver is disposed within the lumen of the implant, as described in reference to Figs. 5D-E, and optionally while using the ultrasound tool or transceiver for imaging).

In the case that additional portions of the wall and/or sites of the tissue are identified as desirable to be anchored (step 220), the delivery tool is steered to another potential anchoring site (step 206). For example, a plurality of tissue anchors 52 can be used to anchor the implant to the tissue, as described in reference to Figs. 2E-K.

In the case that no additional portions of the wall and/or sites of the tissue are identified as desirable to be anchored (step 220), the implant (e.g. implant 20) can be contracted, e.g. by tensioning contraction member 42, e.g. as described in reference to Figs 2L-M.

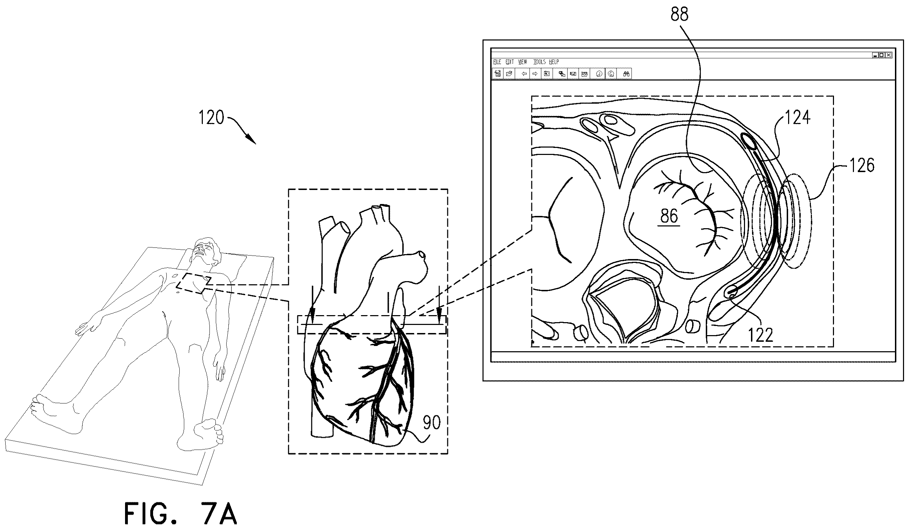

Reference is made to Figs. 7A-B, which are schematic illustrations of an example system 120 that comprises an indicator wire 124 configured for placement in a blood vessel 122 of heart 90 of the subject. Indicator wire 124 can be positioned in a coronary artery or coronary vein (e.g., the coronary sinus, as shown) for applications in which treatment of mitral valve 86 is desired. It is to be noted that indicator wire 124 can be placed within any suitable blood vessel of the heart. As shown, wire 124 is positioned adjacent annulus 88 of valve 86 in order to guide a distal portion of an anchor-delivery system 128 toward the appropriate location along annulus 88 in order to deploy tissue anchor 52 into annulus 88. System 120 provides an indication of the position of the distal end of anchor-delivery system 128 so that an anchor is properly deployed in tissue 92 of annulus 88 and is not inadvertently deployed within tissue of the leaflet or tissue of an atrial wall, or any other undesired tissue.

System 120 is for treating a native valve (e.g., an atrioventricular valve, such as the mitral valve or the tricuspid valve) of heart 90 of the subject. Any and all of the methods, techniques, operations, steps, etc. described herein using system 120 can be performed on a living animal or on a non-living simulation, such as on a cadaver, cadaver heart, simulator (e.g. with the body parts, tissue, etc. being simulated), etc.

Indicator wire 124 can be configured to generate a physical field 126 with respect to wire 124, e.g., surrounding wire 124. For some applications, indicator wire 124 is coupled to an electrical wire which drives an electric current through indicator wire 124. For some applications, field 126 comprises an electric field. For some applications, field 126 comprises an electrostatic field. For some applications, field 126 comprises an electromagnetic field. For some applications, field 126 comprises a magnetic field. Indicator wire 124 is charged with an electrostatic potential or will have current running through it in order to increase its electromagnetic signature.

For some applications, multi-component tubular system 10 described hereinabove comprises anchor-delivery system 128. System 10 is used to deliver and anchor in place implant 20, e.g., an annuloplasty structure, as described hereinabove. The structure comprises a sleeve 30 defining a lumen 48 through which anchor channel 18 of multi-component tubular system 10 passes. A distal end portion of anchor-delivery system 128 comprises the distal end portion of channel 18. The distal end portion of channel 18 comprises a sensing element 130. For some applications, sensing element 130 is ring-shaped, as shown. For some applications, sensing element 130 is shaped to define any other suitable shape. For some applications, sensing element 130 comprises an antenna. For some applications, implant 20 comprises sensing element 130. For some applications, sensing element 130 comprises an ultrasonic sensing element.

For some applications, sensing element 130 wirelessly transmits data relating to physical field 126 to an extracorporeal processing unit and/or monitor (e.g., for analysis, visual display, etc.). For some applications, sensing element is coupled to a wire which transmits the data relating to physical field 126 to an extracorporeal processing unit and/or monitor.

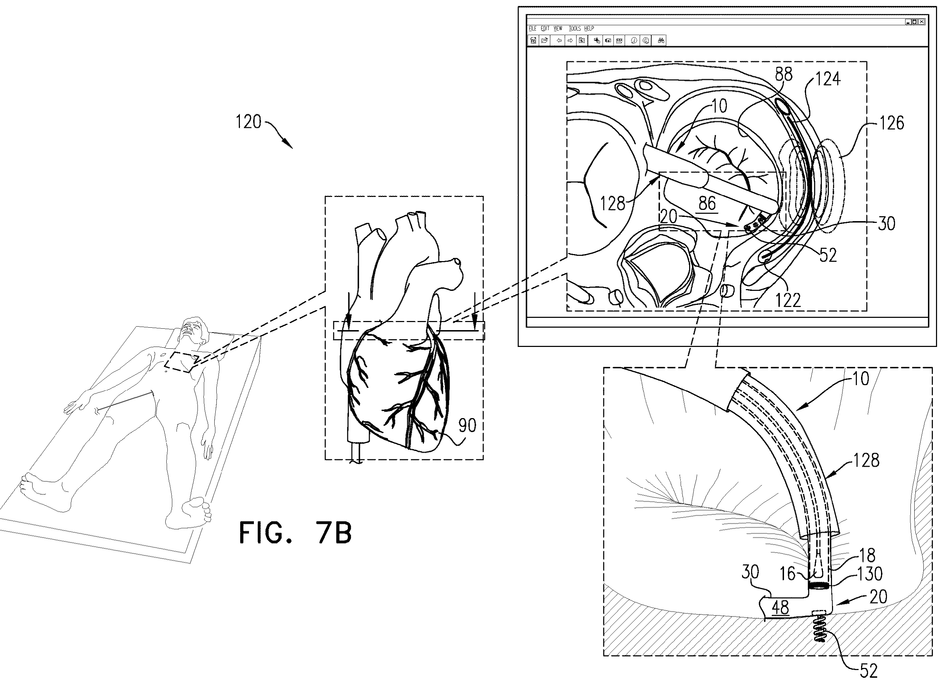

As shown in Fig. 7B, anchor-delivery system 128 delivers tissue anchor 52 toward a portion of tissue 92 of annulus 88 adjacent to blood vessel 122. Anchor driver 16 is used to deliver anchor 52 and to anchor the anchor to tissue 92. Fig. 7B shows anchor 52 following deployment into the tissue. Prior to deployment, the distal portion of anchor delivery system 128, i.e., the distal portion of channel 18 comprising sensing element 130, is brought within range of indicator wire 124 and comes into contact with field 126. That is, the physical field 126 of wire 124 is stationary while channel 18 moves.

The position of distal portion of system 128 is determined by measuring a change in physical field 126 by bringing the portion of anchor-delivery system 128 within range of indicator wire 124 and calculating a distance between the portion of anchor-delivery system 128 and a portion of indicator wire 124. That is, changes in field 126 as shown in Fig. 7B, are viewed, measured, and calculated, and compared with the undistorted field 126 of wire 124 as shown in Fig. 7A. The amount of distortion correlates with the distance between the distal end of channel 18 and wire 124. If the distance is below a predetermined threshold, indicating the appropriate position along annulus 88 (i.e., and not along any undesired tissue such as the leaflet or the atrial wall), then anchor 52 is deployed.

Often, calculating the distance between the portion of anchor-delivery system 128 and the portion of the indicator wire 124 comprises comparing the distance to a predetermined threshold. The predetermined threshold may be a correlation to the amount of distortion in physical field 126. System 120 provides an indication to the operating physician when the proximity between sensing element 130 and wire 124 have reached a predetermined threshold.

Responsively to comparing of the distance to the predetermined threshold, anchor 52 is deployed within tissue 92 of annulus 88. For some applications, tissue anchor 52 is deployed responsively to determining that the distance is below the predetermined threshold. Once anchor 52 is deployed in the proper location along annulus 88, channel 18 of anchor-delivery system 128 is moved to another portion of tissue 92 along annulus 88, and another distortion of field 126 is measured in order to calculate the distance between the distal end portion of channel 18 and wire 124. If the distance is below the threshold, another anchor 52 is deployed within tissue 92 of the annulus.

For some applications, sensing element 130 comprises an ultrasonic sensing element. For applications in which sensing element 130 comprises the ultrasonic sensing element, indicator wire 124 may or may not generate physical field 126. Rather, the ultrasonic sensing element generates an ultrasonic field, and when channel 18 comes within range of indicator wire 124, changes in the ultrasonic field generated by the ultrasonic sensing element due to the proximity of wire 124 are monitored and calculated to determine whether the distance between the distal end of channel 18 and wire 124 is under the predetermined threshold.

That is, the ultrasonic field of channel 18 moves while wire 124 remains stationary.

Reference is again made to Figs. 7A-B. It is to be noted that system 120 can be used to treat the right side of the heart as well. For applications in which implant 20 is placed along the tricuspid valve, wire 124 can be positioned within the right coronary artery.

Reference is again made to Figs. 7A-B. System 120 advantageously provides the operating physician with a better indication as to the location of the portion of anchor-delivery system 128 relative to an artery or vein, thus minimizing or eliminating the possibility of damaging the blood vessel during anchor delivery. As such, system 120 improves subject safety and reduces procedure duration.

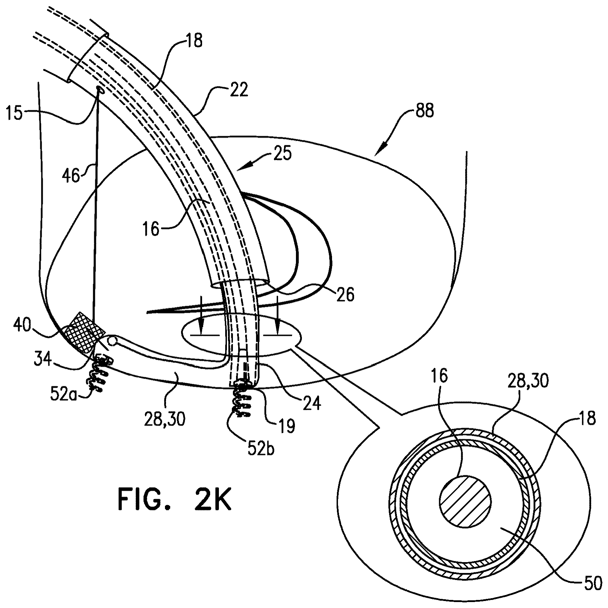

Reference is again made to Figs. 7A-B. For some applications, systems 10 and 120 are used in combination with one or more techniques described in one or more of the following references, which are all incorporated herein by reference for all purposes. Further, the techniques, methods, operations, steps, etc. described or suggested in this disclosure and/or in the following references can be performed on a living animal or on a non-living simulation, such as on a cadaver, cadaver heart, simulator (e.g. with the body parts, tissue, etc. being simulated), etc.
US patent application 12/437,103 to Zipory et al., filed May 7, 2009, which published as US 2010/0286767. For example, (1) systems 10 and 120 of the present application can be used to facilitate the techniques described with reference to Figs. 2-3 and/or 6A-12 of US 2010/0286767 to Zipory et al., mutatis mutandis; (2) anchor driver 16 of the present application can comprise or correspond to anchor driver 68 and/or anchor deployment manipulator 24 of US 2010/0286767 to Zipory et al., mutatis mutandis; (3) tissue anchor 52 of the present application can comprise or correspond to anchor 38 of US 2010/0286767 to Zipory et al., mutatis mutandis; and/or (4) the implant of the present application can comprise or correspond to annuloplasty ring 22 of US 2010/0286767 to Zipory et al., mutatis mutandis.
US patent application 12/689,635 to Zipory et al., filed January 19, 2010, which published as US 2010/0280604. For example, (1) systems 10 and 120 of the present application can be used to facilitate the techniques described with reference to Figs. 2-3 and/or 11A-17 of US 2010/0280604 to Zipory et al., mutatis mutandis; (2) anchor driver 16 of the present application can comprise or correspond to anchor driver 68 and/or anchor deployment manipulator 24 of US 2010/0280604 to Zipory et al., mutatis mutandis; (3) tissue anchor 52 of the present application can comprise or correspond to anchor 38 of US 2010/0280604 to Zipory et al., mutatis mutandis; and/or (4) the implant of the present application can comprise or correspond to annuloplasty ring 22 of US 2010/0280604 to Zipory et al., mutatis mutandis.
PCT patent application IL2012/050451 to Sheps et al., filed November 8, 2013, which published as WO 2013/069019. For example, (1) systems 10 and 120 of the present application can be used to facilitate the techniques described with reference to Figs. 14A-I of WO 2013/069019 to Sheps et al., mutatis mutandis; (2) systems 10 and 120 of the present application can comprise or correspond to system 10 of WO 2013/069019 to Sheps et al., mutatis mutandis; (3) anchor driver 16 of the present application can comprise or correspond to anchor deployment manipulator 61 and/or anchor driver 36 of WO 2013/069019 to Sheps et al., mutatis mutandis; and/or (4) the implant of the present application can comprise or correspond to annuloplasty structure 222 and/or sleeve 26 of WO 2013/069019 to Sheps et al., mutatis mutandis.
PCT patent application IL2013/050860 to Sheps et al., titled "Controlled steering functionality for implant-delivery tool", filed on October 23, 2013, which published as WO 2014/064694. For example, (1) systems 10 and 120 of the present application can be used to facilitate techniques described with reference to Figs. 10A-I, 12A-14B, 18A-C, 21-28, 34, and 36 of this PCT application titled "Controlled steering functionality for implant-delivery tool", mutatis mutandis; (2) systems 10 and 120 of the present application can comprise or correspond to system 10 of this PCT application titled "Controlled steering functionality for implant-delivery tool", mutatis mutandis; (3) anchor driver 16 of the present application can comprise or correspond to anchor deployment manipulator 61, anchor driver 36 and/or deployment element 2338 of this PCT application titled "Controlled steering functionality for implant-delivery tool", mutatis mutandis; and/or (4) the implant of the present application can comprise or correspond to annuloplasty structure 222 and/or sleeve 26 of this PCT application titled "Controlled steering functionality for implant-delivery tool", mutatis mutandis.
PCT patent application IL2013/050861 to Herman et al., titled "Percutaneous tissue anchor techniques", filed on October 23, 2013, which published as WO 2014/064695. For example, (1) systems 10 and 120 of the present application can be used to facilitate the techniques described with reference to Figs. 9A-C and/or 13A-D of this PCT application titled "Percutaneous tissue anchor techniques", mutatis mutandis; (2) tissue anchor 52 of the present application can comprise or correspond to tissue anchor 40 of this PCT application titled "Percutaneous tissue anchor techniques", mutatis mutandis; and/or (3) anchor driver 16 of the present application can comprise or correspond to anchor driver 500, anchor driver 236, deployment manipulator 261, or tool 80 of this PCT application titled "Percutaneous tissue anchor techniques", mutatis mutandis.
PCT patent application IL2019/050777 to Brauon et al., titled "Annuloplasty Systems and Locking Tools Therefor", filed on July 11, 2019, which published as WO 2020/012481.
US provisional patent application 62/949,392 to Kasher et al., titled "Annuloplasty and Tissue Anchor Technologies", filed December 17, 2019.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.
Examples are set out in the following list of numbered clauses:
1. A system for use with a tissue of a subject, the system comprising:
   an implant comprising a wall that surrounds a lumen;
   a delivery tool, the delivery tool comprising:
      a catheter, transluminally advanceable to the tissue and having a distal part that includes a distal opening, the implant configured to be delivered to the tissue via the catheter;
      an extracorporeal control assembly:
         configured to advance at least a portion of the wall out of the distal opening, and
         operably coupled to the distal part to steer the distal part to place the portion of the wall against a site of the tissue, the site disposed distally from the portion of the wall and opposite the distal opening; and
      an ultrasound tool:
         advanceable within the catheter,
         comprising an ultrasound transceiver at a distal end thereof,
         configured to:
            position the ultrasound transceiver in a position that is within the lumen of the implant and facing the portion of the wall, and
            facilitate imaging of the site by transmitting ultrasound energy through the portion of the wall and into the site;
   a tissue anchor; and
   an anchor driver configured to anchor the implant to the tissue by driving the tissue anchor through the portion of the wall and into the site.
2. The system according to clause 1, wherein the catheter is configured to be transfemorally and transseptally advanceable to the tissue.
3. The system according to any one of clauses 1-2, wherein a distal part of the catheter is radiopaque.
4. The system according to any one of clauses 1-3, wherein the anchor driver is configured to advance the tissue anchor through the catheter to the implant while the implant is disposed at the tissue.
5. The system according to any one of clauses 1-4, wherein:
   the ultrasound transceiver is configured to detect reflected ultrasound energy, the reflected ultrasound energy being a portion of the ultrasound energy that is transmitted by the ultrasound transceiver, and that is reflected from the site, and
   the ultrasound tool further comprises:
      an ultrasound controller comprising circuitry and a user interface, the ultrasound controller configured to facilitate analysis of the reflected ultrasound energy detected by the ultrasound transceiver, and
      a connector configured to relay, from the ultrasound transceiver to the ultrasound controller, data indicative of the reflected ultrasound energy detected by the ultrasound transceiver.
6. The system according to clause 5, wherein the ultrasound transceiver is configured to detect reflected ultrasound energy that is reflected from the site through the portion of the wall.
7. The system according to any one of clauses 1-6, wherein the delivery tool further comprises an anchor channel, the anchor channel:
   defining a longitudinal cavity ending at a distal aperture,
   extending through the catheter such that a distal region of the anchor channel is disposed within the lumen, the distal region including the distal aperture, and
   being advanceable within the catheter and the lumen.
8. The system according to clause 7, wherein the anchor channel is configured to extend through the catheter such that:
   a distal region of the anchor channel is disposed within the lumen, the distal region including the distal aperture, and
   the anchor channel facilitates positioning of the portion of the wall at the site.
9. The system according to clause 7, wherein the anchor driver is:
   advanceable, while coupled to the tissue anchor, through the longitudinal cavity to the implant, so as to advance the tissue anchor into the lumen of the implant, in a manner in which the tissue anchor reaches the portion of the wall, and
   removable from the longitudinal cavity, via a proximal opening of the catheter.
10. The system according to clause 9, wherein the ultrasound transceiver is:
   advanceable through the longitudinal cavity into the lumen of the implant such that the ultrasound transceiver is facing the portion of the wall, and
   removable from the longitudinal cavity, through the proximal opening of the catheter.
11. The system according to clause 10, wherein the anchor driver is advanceable through the longitudinal cavity to the implant only while the ultrasound transceiver is not disposed through the longitudinal cavity.
12. The system according to clause 9, wherein:
   the ultrasound tool comprises the anchor channel,
   the ultrasound transceiver is disposed at the distal region of the anchor channel, and
   the anchor channel is configured to position the ultrasound transceiver in a position that is within the lumen of the implant and facing the portion of the wall.
13. The system according to clause 12, wherein the ultrasound transceiver is shaped as a ring, the ring defining a passage therethrough.
14. The system according to clause 13, wherein the anchor driver is advanceable, while coupled to the tissue anchor, through the longitudinal cavity to the implant, so as to advance the tissue anchor through the passage, in a manner in which the tissue anchor reaches the portion of the wall.
15. The system according to any one of clauses 1-14, wherein the implant comprises an annuloplasty structure, the annuloplasty structure comprising a sleeve defined by the wall.
16. The system according to clause 15, wherein the annuloplasty structure further comprises a contraction member and an adjustment mechanism, wherein:
   the adjustment mechanism is configured to, when actuated, apply tension to the contraction member, and
   the contraction member is configured to adjust a length of the annuloplasty structure when the contraction member is tensioned by actuating the adjustment mechanism.
17. A method, comprising:
   placing an indicator wire in a blood vessel of a heart of a non-living simulation;
   using an anchor-delivery system, delivering a tissue anchor toward a portion of tissue of the heart adjacent to the blood vessel, the anchor-delivery system including an ultrasonic sensing element at a portion of the anchor-delivery system; and
   determining a position of the portion of the anchor-delivery system by:
      measuring a change in an ultrasonic field generated by the ultrasonic sensing element by bringing the portion of the anchor-delivery system within range of the indicator wire; and
      by the measuring, calculating a distance between the portion of the anchor-delivery system and a portion of the indicator wire.
18. The method according to clause 17, wherein:
   the portion of tissue comprises tissue of an annulus of the non-living simulation,
   the method further comprises advancing an annuloplasty structure to the annulus, the annuloplasty structure shaped so as to define a lumen therethrough, and
   delivering the tissue anchor to the portion of tissue comprises delivering the tissue anchor from within the lumen of the annuloplasty structure and to a portion of tissue of the annulus.
19. The method according to any one of clauses 17-18, wherein delivering the tissue anchor comprises implanting an annuloplasty structure at an annulus of the heart using the tissue anchor.
20. The method according to clause 19, wherein the annuloplasty structure is shaped so as to define a lumen, and wherein delivering the tissue anchor comprises moving the portion of the anchor-delivery system through the lumen of the annuloplasty structure and deploying the tissue anchor into the portion of tissue from within the lumen of the annuloplasty structure.
21. The method according to any one of clauses 17-20, wherein calculating the distance between the portion of the anchor-delivery system and the portion of the indicator wire comprises comparing the distance to a predetermined threshold.
22. The method according to clause 21, wherein delivering the tissue anchor comprises deploying the tissue anchor into the portion of tissue responsively to the comparing of the distance to a predetermined threshold and/or responsively to determining that the distance is below the predetermined threshold.
23. The method according to clause 22, wherein the tissue anchor defines a first tissue anchor, and wherein the method further comprises:
   subsequently to the deploying of the tissue anchor into the portion of tissue, moving the portion of the anchor-delivery system to a second portion of tissue by determining the position of the portion of the anchor-delivery system; and
   deploying the second tissue anchor into the second portion of tissue responsively to the comparing of the distance to the predetermined threshold.
24. The method according to clause 23, wherein delivering the second tissue anchor comprises deploying the second tissue anchor into the second portion of tissue responsively to determining that the distance is below the predetermined threshold.
25. A method for implanting an implant at tissue in a simulation, the method comprising:
   transluminally advancing the implant or a portion thereof to the tissue using a delivery tool that includes a catheter, the catheter having a distal part that includes a distal opening;
   using a control assembly that is operably coupled to the distal part of the catheter to steer the distal part of the catheter, such that the distal opening is disposed at or near a site of the tissue; and
   positioning an ultrasound transceiver of an ultrasound tool at or near the site of the tissue;
   assessing the site of the tissue, by using the ultrasound tool to:
      transmit ultrasound energy to the site, and
      detect reflected ultrasound energy reflected from the site; and
   subsequently, using an anchor driver, anchoring the implant to the tissue by driving a tissue anchor to the site of the tissue.
26. The method according to clause 25, wherein:
   the tissue includes tissue of an annulus of a heart valve,
   the implant includes an annuloplasty structure having a lumen circumscribed by a wall,
   assessing the site of the tissue comprises using the ultrasound transceiver from inside the lumen of the annuloplasty structure to transmit ultrasound energy through the wall to the site, and
   anchoring the implant to the tissue comprises anchoring a portion of the wall of the annuloplasty structure to the tissue of the annulus.
27. The method according to clause 26, wherein detecting reflected ultrasound energy reflected from the site comprises detecting reflected ultrasound energy reflected back through the portion of the wall.
28. The method according to clause 26, wherein:
   the ultrasound transceiver of the ultrasound tool is disposed at a distal region of the ultrasound tool,
   the portion of the wall is held against the site of the tissue using the ultrasound tool to exert a pushing force against the wall from within the lumen of the implant, and
   driving the tissue anchor comprises driving the tissue anchor through a passage defined by the ultrasound transceiver.
29. The method according to any one of clauses 25-28, wherein the anchor driver is coaxial with the ultrasound tool when anchoring the implant to the tissue.
30. The method according to clause 29, wherein the tissue anchor is coaxial with a distal region of the ultrasound tool when anchoring the implant to the tissue.
31. The method according to any one of clauses 29-30, further comprising using the ultrasound tool and the anchor driver simultaneously while anchoring the implant to the tissue.
32. The method according to any one of clauses 29-30, further comprising continuing to assess the site of the tissue using the ultrasound tool while anchoring the implant to the tissue by driving the tissue anchor into the site of the tissue.
33. The method according to any one of clauses 25-32, wherein:
   the ultrasound tool further includes a connector and an ultrasound controller that includes circuitry and a user interface, and
   the method further comprises:
      using the connector, relaying data indicative of the reflected ultrasound energy detected by the ultrasound transceiver, from the ultrasound transceiver to the ultrasound controller, and
      using the circuitry of the ultrasound controller, analyzing the reflected ultrasound energy.
34. The method according to clause 33, wherein analyzing the reflected ultrasound energy comprises operating a data analysis program in the circuitry.
35. The method according to clause 33, wherein analyzing the reflected ultrasound energy comprises evaluating an image displayed on the user interface.
36. The method according to any one of clauses 25-35, wherein:
   the tissue anchor is a first tissue anchor,
   the site of the tissue is a first site of the tissue,
   driving the tissue anchor into the site of the tissue comprises driving the first tissue anchor into the first site of the tissue, and
   anchoring the implant to the tissue further comprises, subsequently to driving the first tissue anchor into the first site of the tissue, driving a second tissue anchor into a second site of the tissue different from the first site of the tissue.
37. The method according to clause 36, wherein:
   the implant includes a contraction member, and
   the method further comprises adjusting a shape or size of the tissue by tensioning the contraction member.
38. The method according to clause 36, wherein:
   the implant includes an annuloplasty structure having a wall shaped to define a sleeve of the annuloplasty structure, and the annuloplasty structure includes a contraction member extending along at least a portion of the sleeve, and
   the method further comprises adjusting a length of the annuloplasty structure by tensioning the contraction member.
39. The method according to any one of clauses 37-38, wherein tensioning the contraction member comprises tensioning the contraction member by actuating an adjustment mechanism.
40. The method according to clause 39, wherein the adjustment mechanism includes a rotatable spool coupled to the contraction member, and wherein tensioning the contraction member by actuating the adjustment mechanism comprises rotating the spool.
41. The method according to clause 40, further comprising, (i) subsequently to driving the first tissue anchor into the first site of the tissue, and (ii) prior to driving the second tissue anchor into the second site of the tissue:
   steering the distal part of the catheter, such that the distal opening is disposed at or near the second site of the tissue;
   assessing the second site of the tissue, by using the ultrasound tool to:
      transmit ultrasound energy to the second site, and
      detect reflected ultrasound energy reflected from the second site.

## Claims

1. A system for use with a tissue of a subject, the system comprising:
an implant comprising a wall that surrounds a lumen;
a delivery tool, the delivery tool comprising:
a catheter, transluminally advanceable to the tissue and having a distal part that includes a distal opening, the implant configured to be delivered to the tissue via the catheter;
an extracorporeal control assembly:
configured to advance at least a portion of the wall out of the distal opening, and
operably coupled to the distal part to steer the distal part to place the portion of the wall against a site of the tissue, the site disposed distally from the portion of the wall and opposite the distal opening;
an anchor channel defining a longitudinal cavity, and a distal region including a distal aperture; and
an ultrasound tool:
advanceable within the catheter,
comprising an ultrasound transceiver at a distal end thereof, configured to:
position the ultrasound transceiver in a position that is within the lumen of the implant and facing the portion of the wall,
facilitate imaging of the site by transmitting ultrasound energy through the portion of the wall and into the site, and
withdraw the ultrasound transceiver from the delivery tool;
a tissue anchor; and
an anchor driver configured to advance, while coupled to the tissue anchor, through the longitudinal cavity to the site, and to anchor the implant to the tissue by driving the tissue anchor through the portion of the wall and into the site.

2. The system according to claim 1, wherein the anchor channel is configured to hold the portion of the wall against the site of the tissue, and optionally wherein the anchor channel is configured to hold the portion of the wall against the site of the tissue during withdrawal of the ultrasound tool from the delivery tool and the subsequent advancement of the anchor driver through the cavity to the implant.

3. The system according to any preceding claim, wherein the anchor driver and the ultrasound tool are dimensioned such that only one of the anchor driver or the ultrasound transceiver can be disposed within the longitudinal cavity at a given time.

4. The system according to any preceding claim, wherein:
a.) the catheter is configured to be transfemorally and transseptally advanceable to the tissue,
b.) a distal part of the catheter is radiopaque, and/or
c.) the anchor driver is configured to advance the tissue anchor through the catheter to the implant while the implant is disposed at the tissue.

5. The system according to any preceding claim, wherein:
the ultrasound transceiver is configured to detect reflected ultrasound energy, the reflected ultrasound energy being a portion of the ultrasound energy that is transmitted by the ultrasound transceiver, and that is reflected from the site, and
the ultrasound tool further comprises:
an ultrasound controller comprising circuitry and a user interface, the ultrasound controller configured to facilitate analysis of the reflected ultrasound energy detected by the ultrasound transceiver, and
a connector configured to relay, from the ultrasound transceiver to the ultrasound controller, data indicative of the reflected ultrasound energy detected by the ultrasound transceiver, and optionally wherein the ultrasound transceiver is configured to detect reflected ultrasound energy that is reflected from the site through the portion of the wall.

6. The system according to any preceding claim, wherein the anchor channel:
extends through the catheter such that the distal region of the anchor channel is disposed within the lumen, the distal region including the distal aperture, and
is advanceable within the catheter and the lumen.

7. The system according to any preceding claim, wherein:
a.) the anchor channel is configured to extend through the catheter such that:
a distal region of the anchor channel is disposed within the lumen, the distal region including the distal aperture, and
the anchor channel facilitates positioning of the portion of the wall at the site; and/or
b.) the anchor driver is:
advanceable, while coupled to the tissue anchor, through the longitudinal cavity to the implant, so as to advance the tissue anchor into the lumen of the implant, in a manner in which the tissue anchor reaches the portion of the wall, and
removable from the longitudinal cavity, via a proximal opening of the catheter, optionally wherein the ultrasound transducer is:
advanceable through the longitudinal cavity into the lumen of the implant such that the ultrasound transducer is facing the portion of the wall, and
removable from the longitudinal cavity, through the proximal opening of the catheter, further optionally wherein the anchor driver is advanceable through the longitudinal cavity to the implant only while the ultrasound transceiver is not disposed through the longitudinal cavity.

8. The system according to any preceding claim, wherein the implant comprises an annuloplasty structure, the annuloplasty structure comprising a sleeve defined by the wall, optionally wherein the annuloplasty structure further comprises a contraction member and an adjustment mechanism, wherein:
the adjustment mechanism is configured to, when actuated, apply tension to the contraction member, and
the contraction member is configured to adjust a length of the annuloplasty structure when the contraction member is tensioned by actuating the adjustment mechanism.

9. A method for implanting an implant at tissue in a simulation using the system of any preceding claim, the method comprising:
transluminally advancing the implant or a portion thereof to the tissue using a delivery tool that includes a catheter, the catheter having a distal part that includes a distal opening;
using a control assembly that is operably coupled to the distal part of the catheter to steer the distal part of the catheter, such that the distal opening is disposed at or near a site of the tissue; and
positioning an ultrasound transceiver of an ultrasound tool at or near the site of the tissue;
assessing the site of the tissue, by using the ultrasound tool to:
transmit ultrasound energy to the site, and
detect reflected ultrasound energy reflected from the site; and
subsequently, using an anchor driver, anchoring the implant to the tissue by driving a tissue anchor to the site of the tissue.

10. The method according to claim 9, wherein:
the tissue includes tissue of an annulus of a heart valve,
the implant includes an annuloplasty structure having a lumen circumscribed by a wall,
assessing the site of the tissue comprises using the ultrasound transceiver from inside the lumen of the annuloplasty structure to transmit ultrasound energy through the wall to the site, and
anchoring the implant to the tissue comprises anchoring a portion of the wall of the annuloplasty structure to the tissue of the annulus, and wherein, optionally detecting reflected ultrasound energy reflected from the site comprises detecting reflected ultrasound energy reflected back through the portion of the wall.

11. The method according to any one of claims 9-10, wherein:
the ultrasound tool further includes a connector and an ultrasound controller that includes circuitry and a user interface, and
the method further comprises:
using the connector, relaying data indicative of the reflected ultrasound energy detected by the ultrasound transceiver, from the ultrasound transceiver to the ultrasound controller, and
using the circuitry of the ultrasound controller, analyzing the reflected ultrasound energy, and optionally wherein the analyzing the reflected ultrasound energy comprises:
a) operating a data analysis program in the circuitry; or
b) evaluating an image displayed on the user interface.

12. The method according to any one of claims 9-11, wherein:
the tissue anchor is a first tissue anchor,
the site of the tissue is a first site of the tissue,
driving the tissue anchor into the site of the tissue comprises driving the first tissue anchor into the first site of the tissue, and
anchoring the implant to the tissue further comprises, subsequently to driving the first tissue anchor into the first site of the tissue, driving a second tissue anchor into a second site of the tissue different from the first site of the tissue.

13. The method according to claim 12, wherein:
a.) the implant includes a contraction member, and
the method further comprises adjusting a shape or size of the tissue by tensioning the contraction member; or
b.) the implant includes an annuloplasty structure having a wall shaped to define a sleeve of the annuloplasty structure, and the annuloplasty structure includes a contraction member extending along at least a portion of the sleeve, and
the method further comprises adjusting a length of the annuloplasty structure by tensioning the contraction member.

14. The method according to claim 13, wherein tensioning the contraction member comprises tensioning the contraction member by actuating an adjustment mechanism, optionally wherein the adjustment mechanism includes a rotatable spool coupled to the contraction member, and wherein tensioning the contraction member by actuating the adjustment mechanism comprises rotating the spool.

15. The method according to any of claims 12 to 14, further comprising, (i) subsequently to driving the first tissue anchor into the first site of the tissue, and (ii) prior to driving the second tissue anchor into the second site of the tissue:
steering the distal part of the catheter, such that the distal opening is disposed at or near the second site of the tissue;
assessing the second site of the tissue, by using the ultrasound tool to:
transmit ultrasound energy to the second site, and
detect reflected ultrasound energy reflected from the second site.
